(19) Europäisches Patentamt
European Patent Office
Office européen des brevets

(11) EP 2 913 047 B1

# (12) EUROPEAN PATENT SPECIFICATION

(45) Date of publication and mention of the grant of the patent:
**08.05.2019 Bulletin 2019/19**

(21) Application number: **13851236.3**

(22) Date of filing: **28.10.2013**

(51) Int Cl.:
*A61K 9/14* (2006.01) *A61K 9/127* (2006.01)
*A61K 31/137* (2006.01) *A61K 31/343* (2006.01)
*A61K 31/4164* (2006.01) *A61K 31/4174* (2006.01)
*A61K 31/4406* (2006.01) *A61K 31/46* (2006.01)
*A61K 31/47* (2006.01) *A61K 31/4965* (2006.01)
*A61K 31/5578* (2006.01) *A61K 31/5585* (2006.01)
*A61K 31/56* (2006.01) *A61K 31/573* (2006.01)
*A61K 38/00* (2006.01) *A61K 47/34* (2017.01)
*A61K 47/42* (2017.01) *A61K 47/44* (2017.01)
*A61P 9/12* (2006.01) *A61P 11/00* (2006.01)

(86) International application number:
**PCT/JP2013/079134**

(87) International publication number:
**WO 2014/069401 (08.05.2014 Gazette 2014/19)**

## (54) PULMONARY DISEASE-SPECIFIC THERAPEUTIC AGENT

SPEZIFISCHES THERAPEUTIKUM FÜR PULMONALE ERKRANKUNGEN

AGENT THÉRAPEUTIQUE SPÉCIFIQUE DE MALADIE PULMONAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.10.2012 JP 2012238017**

(43) Date of publication of application:
**02.09.2015 Bulletin 2015/36**

(73) Proprietor: **Cardio Incorporated**
**Kobe-shi**
**Hyogo 650-0047 (JP)**

(72) Inventors:
- **SAWA, Yoshiki**
**Suita-shi**
**Osaka 565-0871 (JP)**
- **MIYAGAWA, Shigeru**
**Suita-shi**
**Osaka 565-0871 (JP)**
- **TAIRA, Masaki**
**Suita-shi**
**Osaka 565-0871 (JP)**
- **SAKAI, Yoshiki**
**Sasayama-shi**
**Hyogo 669-2454 (JP)**

(74) Representative: **Vossius & Partner**
**Patentanwälte Rechtsanwälte mbB**
**Siebertstrasse 3**
**81675 München (DE)**

(56) References cited:
**EP-A1- 1 563 846**
**WO-A1-2008/098196**
**WO-A1-2010/135207**
**WO-A1-2012/107364**
**WO-A1-2012/124688**

- **KIMURA YOSUKE ET AL: "A single injection of a sustained-release prostacyclin analog (ONO-1301MS) suppresses airway inflammation and remodeling in a chronic house dust mite-induced asthma model", EUROPEAN JOURNAL OF PHARMACOLOGY, vol. 721, no. 1, 12 October 2013 (2013-10-12), pages 80-85, XP028789358, ISSN: 0014-2999, DOI: 10.1016/J.EJPHAR.2013.09.051**
- **MASAKI TAIRA ET AL: "Novel Synthetic Prostacyclin Agonist, Ono1301, Inhibits Proliferation of Pulmonary Arterial Smooth Muscle Cells by Suppressing Raf Kinase Signal Pathway", CIRCULATION, vol. 126, no. Suppl., 2 November 2012 (2012-11-02), page A17802, XP055252945, US ISSN: 0009-7322**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 913 047 B1

## Description

### Technical Field

The present invention relates to a pulmonary-disease-specific therapeutic agent.

### Background Art

Generally, inhalants (preparations for transpulmonary administration) have been developed and investigated as a method for lung-specific administration of agents for treating pulmonary diseases. Inhalants have been used as an administration method that is expected to provide local effects to the lungs while avoiding systemic side effects. In the development of an inhalant as a lung-specific administration method, many problems in terms of pharmaceutical preparation, particle size, irritancy, drug metabolism, physical properties, etc., remain to be solved to continuously deliver a pharmaceutical compound to the lungs and maintain its amount in the lungs.

There are also problems for target patients, such as difficulty in handling inhalers, frequent inhalation (persistence), reduction of adherence, weak inhalation strength of pulmonary disease patients, airway obstruction due to sputum, difficulty in delivery of pharmaceutical compounds to the target site due to bronchiole obstruction induced by a lesion in a peripheral, and necessity of removing the drug remaining in the oral cavity by gargling or the like in order to avoid side effects.

Therefore, an inhalant that is effective as a lung-specific administration method is often difficult to develop.

In the past, aerosols were common as inhalants. However, since the use of chlorofluorocarbons for spraying has been prohibited, powder inhalants (DPI: dry powder inhalation) that use no gas have mainly been used. Since a pharmaceutical powder alone adheres to or remains in instruments, secondary particles must be formed with an inert carrier, such as lactose, to reduce adhesion and facilitate inhalation. As inhalants for treating asthma, corticosteroids (steroids), combinations of a corticosteroid with a β2 stimulant (LABA), etc., are mainly used at present. When an inhaled steroid is used, it is necessary to gargle after inhalation to remove the unnecessary drug remaining in the mouth and throat in order to prevent infectious diseases. Diskus inhalers, in which a tablet or capsule containing a pharmaceutical powder is set, are also often used. However, it is often difficult for the elderly and children to handle Diskus inhalers.

On the other hand, systemic dosage forms such as oral preparations, patches, and intravenous injections have a problem of divergence of efficacy from side effects because elevated blood levels of a pharmaceutical substance may cause systemic side effects.

Examples of pulmonary diseases include acute pneumonia, pulmonary fibrosis, interstitial pneumonia, pulmonary hypertension, chronic obstructive pulmonary disease (COPD), chronic bronchitis, pulmonary emphysema, asthma, refractory asthma, systemic inflammatory response syndrome (SIRS), acute lung injury (ALI), acute respiratory distress syndrome (ARDS), sarcoidosis, chronic idiopathic pulmonary thromboembolism, diffuse panbronchiolitis, cystic fibrosis, hypersensitivity pneumonitis, lung cancer, obesity hypoventilation syndrome, alveolar hypoventilation syndrome, and chronic rejection in lung transplantation. Particularly important diseases are pulmonary fibrosis, interstitial pneumonia, pulmonary hypertension, asthma, COPD, and SIRS.

For these diseases, inhalants, patches, oral preparations, intravenous infusions, etc., have been developed. However, sufficiently effective pharmaceutical preparations have yet to be developed.

The current status and problems of typical pulmonary-disease-therapeutic drugs are described below.

1. Asthma and Chronic Obstructive Pulmonary Disease (COPD)

The Japan domestic market size of respiratory disease therapeutic agents is 302.1 billion yen (2009), about 60% of which is agents for treating asthma. The market size of chronic obstructive pulmonary disease (COPD) is 33.2 billion yen. Although the number of potential COPD patients is said to be approximately 5.3 million, the number of patients who have actually received treatment is less than 10% at present. Expansion of the market size is foreseen with an increasing number of patients in the future.

The number of asthma patients in Japan is 888,000. The number of asthma patients is 69.88 per 10,000 population (2008) and is increasing each year. Steroids, β2 receptor agonists, leukotriene receptor antagonists (LTRAs), anticholinergic drugs, theophylline preparations, etc., are used as asthma therapies. In asthma therapy, steroids are used as a first-line drug because airway inflammation must be suppressed. In view of side effects and the like, steroids are usually used in the form of inhalants (pulmonary dosage preparations). Examples of inhaled steroids include fluticasone (trade name: Flonase) and beclomethasone (trade name: Qvar). Inhalants (trade names: Adoair, Symbicort, etc.) comprising a long-acting β2 agonist (LABA) (e.g., salmeterol) and a steroid drug are also used. However, inhalants also cause problems, such as difficulty in handling inhalers, frequent inhalation, reduction of adherence, weak inhalation strength of pulmonary disease patients, airway obstruction due to sputum, and difficulty in delivery of pharmaceutical

compounds to the target site due to sputum. It is thus often difficult to effectively use inhalants.

For children and the elderly who have difficulty in using inhalants, leukotriene receptor antagonists (LTRA) are used as oral agents for treating asthma. The use of LTRA is recommended for remodeling prevention and amelioration, exercise-induced asthma, aspirin asthma, and the like. Examples of typical LTRAs include pranlukast (trade name: Onon) and montelukast (trade name: Singulair). However, since there is asthma that is not mediated by leukotriene, LTRAs are ineffective for about 40% of patients.

Further, among β2-adrenergic stimulants, examples of inhalants include salmeterol (trade name: Serevent), patches such as tulobuterol (trade name: Hokunalin tape), oral preparations such as procaterol (trade name: Meptin), clenbuterol (trade name: Spiropent), formoterol (trade name: Atock), and the like. Other examples include theophylline (trade name: Theo-Dur), which is a phosphodiesterase (PDE) inhibitor that causes relaxation of bronchial smooth muscle, and anticholinergic drugs (M3 receptor antagonists) that inhibit bronchial smooth muscle contraction, such as ipratropium (trade name: Atrovent). However, there is no pharmaceutical compound or drug that provides effects better than steroids. Anticholinergic drugs are contraindicated in patients with glaucoma, prostatic hypertrophy, or the like.

WHO estimates that while the worldwide number of patients with moderate to more severe levels of chronic obstructive pulmonary disease (COPD) in 2005 was 80 million, and COPD causes three million deaths per year worldwide and is the fourth leading cause of death, COPD-related deaths will increase by 30% in the next 10 years. According to the statistics of the Ministry of Health, Labor and Welfare in Japan, the number of patients who presented to a hospital with COPD was 340,000 in 2001, the number of potential patients was 5,300,000 (in 2004), and COPD caused 14,416 deaths (1.3% of total mortality)(in 2005) and is the tenth leading cause of death. In men, COPD is the seventh leading cause of death.

The main drug therapy for COPD is bronchodilators (e.g., anticholinergic drugs, β2 stimulants, and theophylline drugs). Inhalants are recommended in view of efficacy and side effects. Long-acting inhaled anticholinergic drugs and inhaled β2 stimulants are mainly used. When airflow obstruction is severe and multiple exacerbations occur, a combination of a long-acting β2-stimulant and an inhaled steroid is used.

As an anticholinergic drug (M3 antagonist), tiotropium (trade name: Spiriva), which is a first-line inhalant for treating COPD, is used. Tiotropium is superior to long-acting β2-stimulants in terms of bronchodilation and symptom alleviation. However, tiotropium is contraindicated in patients with dysuria due to prostatic hypertrophy, and in glaucoma patients. The major side effects are dry mouth, palpitations, nausea, dysuria, ileus, etc.

A tulobuterol tape (trade name: Hokunalin tape), which is a patch of a β2 agonist, is inferior to inhalants in terms of bronchodilation, but is a pharmaceutical preparation that is easily applicable to the elderly who have difficulty in inhaling. The side effects include palpitations, tremors, headache, nausea, and vomiting.

Theophylline, which is a PDE inhibitor, is inferior to inhalants in terms of bronchodilation for COPD. In recent years, anti-inflammatory effects of theophylline on the airways have attracted attention, and theophylline is expected to be useful as a therapeutic drug. However, since its effective blood concentration range is narrow, the appropriate blood concentration must be monitored.

These are all pharmaceutical compounds or preparations for dilating bronchioles and transiently improving pulmonary function. There are no pharmaceutical compounds or preparations that inhibit destruction of pulmonary alveoli or regenerate the pulmonary alveoli.

2. Interstitial Pneumonia (Pulmonary Fibrosis)

Interstitial pneumonia (pulmonary fibrosis) has been designated as an intractable disease and is a disease with high mortality. However, there are no effective pharmaceutical compounds or preparations for interstitial pneumonia. Pirfenidone (trade name: Pirespa) was released in 2008 but has been approved only in Japan and develops side effects such as photodermatosis and impaired liver function. For idiopathic pulmonary fibrosis (usual interstitial pneumonia in the histopathological tissue classification), only this drug has been on the market. However, its efficacy is said to be insufficient.

3. Pulmonary Hypertension

Pulmonary arterial hypertension has been designated as an intractable disease. According to statistics compiled by the Japanese Ministry of Health, Labor and Welfare in 2009, the number of patients with pulmonary arterial hypertension (idiopathic and hereditary pulmonary arterial hypertension) in Japan is increasing every year, and was reported to be 1,140 in 2008. About 100 people develop pulmonary hypertension each year. Further, collagen disease (e.g., rheumatoid arthritis) is reported to accompany pulmonary hypertension.

A therapeutic method is selected according to the severity and pathological conditions of pulmonary hypertension. Examples of effective drugs include continuous intravenous infusions such as epoprostenol sodium (sodium $PGI_2$, trade name: Flolan). Epoprostenol sodium is used for severe pulmonary hypertension and is the most effective of currently

available therapeutic drugs. However, because this drug has a very short half-life of about 6 minutes, the pharmaceutical preparation must be continuously administered into the body via a central venous catheter. Other $PGI_2$ derivative preparations (carbacyclin derivatives) in the form of inhalants and subcutaneous injections are also available outside of Japan, but side effects develop, such as blood pressure decrease, headache, hot flashes, jaw pain, diarrhea, nausea, and rash. Further, as oral preparations, a PDEV inhibitor (Sildenafil), an ET-1 antagonist (Bozentan), Beraprost, which is a $PGI_2$ derivative, and the like are also available, but these are indicated in mild to moderate patients.

4. SIRS

Systemic inflammatory response syndrome (SIRS) is a critical disease in that it may develop into multiple organ failure (MOF). However, no effective therapeutic agents for SIRS are currently available. Glucocorticoid steroids and a neutrophil elastase inhibitor (sivelestat, trade name: Elaspol) can be used. Since MOF often has a fatal outcome, development into multiple organ failure must be prevented by providing intensive care at the stage of SIRS.

Novel drugs targeting these intractable pulmonary diseases are being developed worldwide. Since the prohibition on use of chlorofluorocarbons, there has been a desire to develop powder inhalants and inhalers as well as a more effective lung-specific therapeutic method due to weak inhalation strength of patients with a respiratory disorder.

Accordingly, oral preparations, intravenous injections, and transdermal administration agents have been developed. However, these pharmaceutical preparations often have insufficient divergence from systemic side effects resulting from systemic administration.

The present inventors previously studied pharmacological effects of prostaglandins (PGs). During the study, the inventors noticed similarities in action between prostaglandins and in vivo regeneration factors, and thus focused on and investigated the possibility that these PGs might induce the production of various in vivo regeneration factors. As a result of the investigation, the inventors found that among prostaglandins (PGs), agonists of IP receptor ($PGI_2$), $EP_2$ receptor, and $EP_4$ receptor, which promote production of cyclic AMP (cAMP), promote lumen formation when normal human umbilical vein vascular endothelial cells (HUVEC) and normal human dermal fibroblasts (NHDF) are co-cultured. As a result of further research, the inventors found that these agonists induce various in vivo regeneration factors from fibroblasts, smooth muscle cells, vascular endothelial cells, and macrophages. As result of further research, the inventors selected, as an inducer of an in vivo regeneration factor, a prostaglandin (PG) I type (IP receptor) that is mainly biosynthesized in vascular endothelial cells, and selected (E)-[5-[2-[1-phenyl-1-(3-pyridyl)methylideneaminooxy]ethyl]-7,8-dihydronaphthalen-1-yloxy]acetic acid (hereinafter sometimes referred to as "Compound 1") as a selective agonist of IP receptor, which is an oxime (OX) derivative not having a prostaglandin (PG) skeleton and which also has thromboxane (TX) $A_2$ synthase inhibitory activity (Patent Literature (PTL) 2).

Prostaglandins (PGs) are classified as autacoids. Prostaglandins (PGs) are synthesized in a required amount at a site requiring PGs when they are required, and are quickly metabolically deactivated topically after manifestation of the action. Accordingly, unlike hormones, prostaglandins (PGs) are not systemically circulated. In clinical application, prostaglandins (PGs) had problems such as a short half-life in blood due to scientific instability. In systemic administration injectable preparations of prostaglandins (PGs) had problems such as hypotensive action associated with vasodilation, headache, and flushing; and oral preparations thereof had problems such as induction of diarrhea and intussusception in addition to hypotensive action. To avoid these problems, the present inventors investigated the possibility of directly administering a long-acting pharmaceutical preparation to the site of disease.

The present inventors thought that sustained release of Compound 1 to an ischemic site requiring angiogenesis or to an injured site requiring tissue repair would be able to continuously induce the production of various in vivo regeneration factors in the periphery of the injured site, in addition to vasodilating the remaining blood vessels at the ischemic site and increasing blood flow based on platelet aggregation inhibitory action, thus providing a DDS (drug delivery system) preparation that has fewer side effects than systemic administration. The inventors further thought that a medicament with fewer side effects than systemic administration and with improved administration compliance of less administration frequency could be created if a pharmaceutical preparation that can release Compound 1 in a sustained manner during the period until angiogenesis (regeneration) or tissue repair has occurred at the ischemic site or in the periphery of the injured tissue site.

The present inventors conducted extensive research and arrived at the idea of producing a microsphere (MS) preparation comprising Compound 1 and a lactic acid-glycolic acid copolymer (PLGA) (hereinafter sometimes referred to as "Compound 1/MS") from the structural features of Compound 1. Compound 1/MS is formulated and designed in such a manner that the the PLGA is hydrolyzed into lactic acid and glycolic acid at the administration site and Compound 1 contained in the microspheres is released almost linearly to the living body. Sustained-release preparations that can release the active ingredient for 1 week to 6 months through the adjustment of the molecular weight of PLGA, the ratio of lactic acid to glycolic acid, and particle size have been developed so far (Patent Literature (PTL) 1 and 2).

As mentioned above,when Compound 1/MS is subcutaneously administered or intramuscularly injected, Compound 1/MS can be used as a long-term intravenous infusion or like preparation.

An investigation of pharmacological effects of Compound 1 revealed that when fibroblasts or smooth muscle cells are cultured with Compound 1, Compound 1 promotes the production of various in vivo regeneration factors and repairs tissue due to anti-apoptosis action, neovascularisation promoting action, stem-cell differentiation-inducing action, anti-fibrotic action, etc. Examples of in vivo regeneration factors include hepatocyte growth factor (HGF), vascular endothelial cell growth factor (VEGF), epidermal growth factor (EGF), insulin-like growth factor (IGF-1), stromal cell-derived factor (SDF-1), various fibroblast growth factors (a/b-FGF), transformation growth factor-β (TGF-β), platelet-derived growth factor (PDGF), angiopoietin, hypoxia-inducible factor (HIF-1), bone morphogenetic protein (BMP), connective tissue growth factor (CTGF), nerve growth factor (NGF), brain-derived neurotrophic factor (BDNF), glia-cell-derived neurotrophic factor (GDNF), stem cell factor (SCF), granulocyte-colony-stimulating factor (G-CSF), keratinocyte growth factor (KGF), chondrocyte growth factor (GDF), leukemia inhibitory factor (LIF), HMGB1, and Kruppel-like transcription factors (KLF), and growth factors of their families.

Examples of in vivo regeneration factors further include extracellular matrixes (e.g., fibronectins, laminins, and proteoglycans) and cell adhesion factors (e.g., cadherins and integrins).

The in vivo regeneration factor is preferably, for example, at least one member selected from the group consisting of b-FGF, HGF, VEGF, EGF, SDF-1, IGF-1, LIF, HIF-1, HMGB-1, G-CSF, and extracellular matrices.

Due to these pharmacological actions, Compound 1 is known to be useful as an agent for preventing and/or treating various organ injuries, such as heart diseases (e.g., myocardial infarction, angina, ventricular tachyarrhythmia, congestive heart failure, diastolic dysfunction, idiopathic cardiomyopathy, dilated cardiomyopathy, atrial fibrillation, myocarditis, and chronic rejection in heart transplantation, liver diseases (e.g., fulminant hepatitis, acute hepatitis, chronic hepatitis, liver cirrhosis, fatty liver, and liver transplantation), renal diseases (e.g., acute kidney injury (AKI), chronic kidney disease (CKD), glomerulonephritis, nephrosclerosis, nephropathy in dialysis patients, ischemic renal damage, tubular transport abnormalities, Cushing's syndrome, and tubulointerstitial disease), pulmonary diseases (e.g., acute pneumonia, pulmonary fibrosis, pulmonary hypertension, chronic obstructive pulmonary disease (COPD), asthma, refractory asthma, systemic inflammatory response syndrome (SIRS), acute lung injury (ALI), acute respiratory distress syndrome (ARDS), sarcoidosis, interstitial pneumonia, and hypersensitivity pneumonitis), pancreatic diseases (e.g., diabetes and chronic pancreatitis), bone diseases (e.g., osteoarthritis, rheumatoid arthritis, osteoporosis, bone fracture, osteonecrosis, and periosteal damage), diabetic complications (e.g., neuropathy, skin ulcer, and nephropathy), dental diseases (e.g., periodontal disease, tooth extraction wounds, oral wounds, and periodontal tissue disorders), neurological diseases (e.g., diabetic neuropathy, spinal stenosis, spinal cord injury, and amyotrophic lateral sclerosis (ALS)), skin ulcers, bedsores, hair loss, etc., and organ/tissue transplants (e.g., liver transplants, kidney transplants, lung transplants, pancreatic islet transplants, and pancreas transplants). This is disclosed in detail, for example, in WO2004/032965 and WO2008/0478631.

Compound 1 was also found to activate cAMP/PKA to inhibit phosphorylation of ERK1/2, which has progressed with the pathological conditions, and inhibit migration, multiplication, and collagen production of fibroblasts (Non-patent Literature (NPL) 1).

Compound 1 has cytoprotective effects due to antiapoptotic action and antinecrotic action. These protective effects were attenuated by administering an anti-HGF neutralizing antibody (Non-patent Literature (NPL) 2). Various pharmacological effects of Compound 1 are disclosed in documents (Non-patent Literature (NPL) 3 and Non-patent Literature (NPL) 4).

The present inventors have already found various effects of Compound 1 on intractable pulmonary diseases.

HGF was found by Nakamura et al. in 1984 (Non-patent Literature (NPL) 5). Later, many researchers proved that HGF plays an important role not only in the liver but also in the lungs, in tissue repair as a growth factor for pulmonary alveolar capillaries and alveolar epithelial cells after lung injury. HGF is also demonstrated to have effects on pulmonary alveolar construction in the fetal period and on small airway epithelial cells (Non-patent Literature (NPL) 6).

These results revealed that HGF can prevent or treat acute or chronic inflammation of bronchiolar epithelial cells and lung cell injury caused by oxidative stress. It is thus suggested that HGF is effective for adult respiratory distress syndrome (ARDS), which is an acute lung injury (Non-patent Literature (NPL) 7), systemic inflammatory response syndrome (SIRS) and pulmonary hypertension (Non-patent Literature (NPL) 8), chronic obstructive pulmonary disease (COPD) (Non-patent Literature (NPL) 9 and Non-patent Literature (NPL) 10), refractory asthma (Non-patent Literature (NPL) 11), and the like.

Further, HGF was proved to exert potent anti-fibrotic effects by inhibiting expression of transforming growth factor-β (TGF-β) that is important for pathogenesis of chronic fibrotic diseases (Non-patent Literature (NPL) 12). It has been suggested that HGF is also effective as an anti-fibrotic agent for pulmonary fibrosis (Non-patent Literature (NPL) 13), etc.

Further, because Compound 1 promotes the production of HGF and the like from fibroblasts, smooth muscle cells, vascular endothelial cells, macrophages, etc., Compound 1 has great potential to be clinically applied to pulmonary hypertension, pulmonary fibrosis, chronic obstructive pulmonary disease (COPD), (intractable) asthma, etc.

The first-line drug (WHO-FCIV) in the treatment of severe pulmonary arterial hypertension (PAH) is an epoprostenol (sodium PGI2 intravenous infusion). However, epoprostenol is a 24-hour intravenous infusion, and continuous administration attenuates its effects (develops tolerance), thus requiring an increase in dose. The efficacy of Compound 1 in terms of inhibiting pulmonary artery media thickening, suppressing pulmonary artery pressure, inhibiting right ventricular hypertrophy, and prolonging survival, as well as inhibiting tolerance development, was confirmed through repetitive subcutaneous administration of Compound 1 (Non-patent Literature (NPL) 14), repetitive oral administration of Compound 1 (Non-patent Literature (NPL) 15), and intermittent subcutaneous administration of Compound 1/MS (Non-patent Literature (NPL) 16) to monocrotaline-induced rat pulmonary hypertension models.

For pulmonary fibrosis, only pirfenidone has been approved in Japan, and its clinical efficacy is still insufficient. The efficacy of Compound 1 in terms of lung weight, lung fibrosis, lung hydroxyproline content, prolongation of survival, etc., was confirmed through repetitive subcutaneous administration (Non-patent Literature (NPL) 17) and repetitive oral administration (Non-patent Literature (NPL) 18) of unmodified Compound 1, and intermittent subcutaneous administration of Compound 1/MS to bleomycin-induced pulmonary fibrosis models.

The efficacy of Compound 1 in terms of alveolar destruction inhibition, respiratory function improvement, etc., was confirmed through single subcutaneous administration of Compound 1/MA to models of COPD induced by cigarette smoke solution (see Pharmacological Effect Test 4 below).

With respect to asthma, inhibition of airway hypersensitivity, goblet cell hyperplasia, submucous fibrosis, and hyperplasia of bronchial smooth muscle cells was confirmed by using acute, chronic, and intractable OVA-induced asthma models (Non-patent Literature (NPL) 19 and Non-patent Literature (NPL) 20), and mite-induced asthma models (Non-patent Literature (NPL) 21 and Non-patent Literature (NPL) 22).

Even when Compound 1 was therapeutically administered after the onset of symptoms, these efficacies were exhibited, thus confirming reverse remodeling effects. These effects were attenuated or eliminated by administration of an anti-HGF neutralizing antibody or an IP receptor antagonist (CAY10449).

The present inventors investigated sustained-release preparation (microspheres (MS)) of Compound 1 and have already developed microsphere preparations (compound 1/MS) using compound 1 and a biodegradable polymer selected from various lactic acid polymers (PLA), glycolic acid polymers (PGA), and lactic acid-glycolic acid copolymers (PLGA) as preparations exhibiting long-term intravenous infusion-like blood kinetics by subcutaneous administration or intramuscular administration of about once a week to about once every six months (PTL 1, Japanese Patent Application No. 2012-208799 (unpublished)). Examples of such polymers include PLA0005, PLA0010, PLA0020, PLA0050, PGA0005, PGA0010, PGA0020, PGA0050, PLGA7520, PLGA7550, PLGA5020, and PLGA5050 (5-50) (Wako Pure Chemical Industries, Ltd., and Mitsui Chemicals, Inc.).

"PLA0005" refers to a lactic acid polymer having a weight average molecular weight of 5,000. "PLA0010" refers to a lactic acid polymer having a weight average molecular weight of 10,000. "PLA0050" refers to a lactic acid polymer having a weight average molecular weight of 50,000.

Similarly, "PGA0005" refers to a glycolic acid polymer having a weight average molecular weight of 5,000. "PGA0050" refers to a glycolic acid polymer having a weight average molecular weight of 50,000. "PLGA7520" refers to a copolymer comprising 75 mol% of lactic acid and 25 mol% of glycolic acid and having a weight average molecular weight of 20,000. "PLGA7550" refers to a copolymer comprising 75 mol% of lactic acid and 25 mol% of glycolic acid and having a weight average molecular weight of 50,000.

A mixture of one to five types of microsphere (MS) preparations prepared by using a polylactic acid (PLA), a lactic acid-glycolic acid copolymer (PLGA), or a polyglycolic acid (PGA) having a weight average molecular weight of 1,000 to 50,000 may be preferable as a biodegradable polymer. For example, when Compound 1 is formulated into a microsphere (MS) preparation using PLA0020, an approximately 16-week sustained-release MS preparation is obtained.

On the other hand, various pharmaceutical compounds containing Compound 1, protein preparations such as enzymatically unstable hormones or in vivo regeneration factors, and peptide preparations containing their active sites can be formed into sustained-release microsphere (MS) preparations of various drugs by using the biodegradable polymers mentioned above or other materials such as gelatin hydrogels (PTL 3 to 6), liposomes or lipids (e.g., PTL 7 and NPL 23). Examples of pharmaceutical compounds include proteins such as various growth factors, and specific examples include basic fibroblast growth factors (b-FGF) and other FGFs, vascular endothelial growth factors (VEGF), hepatocyte growth factors (HGF), platelet-derived growth factors (PDGF), transforming growth factors (TGF), and angiopoietin and like growth factors, hypoxia-inducible factors (HIF), various cytokines, chemokines, adrenomedulin, and extracellular matrices.

For example, PTL 3 discloses producing a sustained-release gelatin preparation of b-FGF by the following process. After 10 wt% acidic gelatin is chemically crosslinked with glutaraldehyde, the crosslinking agent is inactivated, followed by washing with distilled water several times to obtain a crosslinked gelatin hydrogel with a water content of 90.3%. Subsequently, a 0.05M phosphate buffer containing 100 $\mu$g of b-FGF (pH 7.4, PBS 500 $\mu$l) was added dropwise to impregnate the gelatin hydrogel with b-FGF, thus obtaining a microsphere (MS) preparation of b-FGF-impregnated gelatin hydrogel. This MS preparation is disclosed as a pharmaceutical composition for treating coronary artery narrowing or obstruction.

The average particle size of the obtained gelatin hydrogel particles varies according to the concentration of gelatin, the volume ratio of the gelatin aqueous solution to olive oil, and stirring speed, etc, when the particles are produced. The particle size is typically 1 to 1,000 μm. Particles of the required size may be appropriately screened and used depending on the purpose.

This process can produce sustained-release preparations of various growth factors, cytokines, monokines, lymphokine, other bioactive substances, and like bioabsorbable polymer hydrogels (Non-patent literature (NPL) 24 to 26).

The "gelatin hydrogel" refers to a hydrogel obtained by forming a chemical crosslinkage between any of a variety of chemical crosslinking agents and a gelatin molecule by using the gelatin mentioned above. Examples of chemical crosslinking agents include glutaraldehyde, water-soluble carbodiimides such as EDC, propyleneoxide, a diepoxy compound, and condensing agents that form a chemical bond between a hydroxyl group, a carboxyl group, an amino group, a thiol group, an imidazole group, or the like. Glutaraldehyde is preferred. Gelatin can also be chemically crosslinked by a heat treatment or ultraviolet irradiation. These crosslinking treatments may be used in combination. Alternatively, a hydrogel can be also produced by physical crosslinking utilizing salt crosslinking, electrostatic interaction, hydrogen bonds, hydrophobic interaction, or the like.

For example, when b-FGF is immobilized on a gelatin hydrogel, almost no b-FGF is released from the hydrogel. As the hydrogel degrades in vivo, gelatin molecules become water-soluble and are released from b-FGF immobilized on the gelatin molecules. Specifically, sustained release of b-FGF can be controlled by degradation of the hydrogel. Degradability of the hydrogel can be changed by adjusting the degree of crosslinking during the production of the hydrogel. Interaction of b-FGF with gelatin enhances the stability of b-FGF and gelatin in vivo, such as enzymolysis resistance.

A "liposomal preparation" refers to cell-like microparticles comprising phospholipids and glycolipids constructing a biomembrane, and is used as a sustained-release drug-delivery system (DDS) preparation by encapsulating a water-soluble or lipid-soluble pharmaceutical compound into the microparticles.

Examples of phospholipids that can be used include glycerophospholipids such as phosphatidylcholine, phosphatidylethanolamine, phosphatidylseline, phosphatidic acid, phosphatidylglycerol, phosphatidylinositol, cardiolipin, yolk lecithin, hydrogenated yolk lecithin, soy lecithin, and hydrogenated soy lecithin; sphingophospholipids such as sphingomyelin, ceramide phosphorylethanolamine, and ceramide phosphorylglycerol; and plasmalogens.

Examples of glycolipids include glycerolipids such as digalactosyl diglyceride and galactosyl diglyceride sulfate; and sphingoglycolipids such as galactosylceramide, galactosylceramide sulfate, lactosylceramide, ganglioside G7, ganglioside G6, and ganglioside G4.

Examples of sustained-release preparations include genes that encode in vivo regeneration factors (e.g., HGF, b-FGF, SDF-1, HMGB-1, LIF, and HIF-1).

Patent Literature (PTL) 8 and Non-patent Literature (NPL) 27 disclose a method for producing a sustained-release preparation of rapamycin or simvastatin, which are poorly water-soluble substances, and disclose that the sustained-release preparation can be produced by using a gelatin derivative having a hydrophobic group added thereto. The "gelatin derivative having a hydrophobic group added thereto" refers to a gelatin derivative prepared by covalently bonding a hydrophobic group to a gelatin molecule. Examples of hydrophobic groups include polyesters such as polylactic acids (PLA), polyglycolic acids (PGA), and poly-ε-caprolactone, lipids such as cholesterol and phosphatidylethanolamine, aromatic or heteroaromatic groups containing alkyl groups and benzene rings, and mixtures thereof.

Patent Literature (PTL) 9 discloses a biodegradable particle inhalant or intravenous infusion for pulmonary diseases mainly consisting of an anticancer therapy drug.

PTL 9 does not disclose a microsphere (MS) preparation having a number average particle size of 20 μm (20,000 nm) to 50 μm (50,000 nm).

Patent Literature (PTL) 10 discloses that a sustained-release preparation of b-FGF and HGF protein is prepared by using a gelatin hydrogel and that oral or parenteral administration of the sustained-release preparation is effective for pulmonary hypertension. However, the average particle size of gelatin hydrogel particles is 1 to 1,000 μm, and the particle size is not particularly limited. As the administration method, only intraperitoneal administration is disclosed in the Examples. That is, PTL 10 merely discloses a method of use for sustained release of a protein in vivo and is not directed to a method for lung-tissue-specific (DDS) administration.

Patent Literature (PTL) 11 discloses that transpulmonary administration of a sustained-release gelatin hydrogel preparation prepared using b-FGF, HGF, or the like is effective for pulmonary emphysema. PTL 11 discloses that the preparation preferably has a particle size of about 0.1 to 20 μm. PLT 11 does not disclose any specific example of a preferable particle size (20 to 40 μm). The preparation disclosed in PTL 11 is used for transpulmonary administration.

Non-patent literature (NPL) 28 discloses that pulmonary artery administration of sustained-release bFGF gelatin microspheres is effective for pulmonary emphysema. However, NPL 28 does not disclose any limitation of the particle size. Furthermore, artery administration requires skilled medical professionals.

As shown above, there has been no patent or like report on pulmonary-disease-selective therapy by intermittent intravenous administration of a sustained-release microsphere (MS) preparation (in particular, a sustained-release MS

preparation having a number average particle size of 20 to 40 μm).

The inventors prepared a microsphere (MS) preparation using a low molecular weight compound such as Compound 1 and a lactic acid-glycolic acid copolymer (PLGA), and basically reported its efficacy in the form of a preparation for topical administration (for ASO, myocardial infarction, dilated cardiomyopathy, diabetic neuropathy, etc.) and a subcutaneous preparation (for chronic kidney disease,chronic phase of cerebral infarction, asthma, COPD, pulmonary hypertension, pulmonary fibrosis, etc.) that exhibits intravenous infusion-like blood kinetics (PTL 2 and PTL 1) . However, no intravenous administration method for lung-specific delivery and maintenance is disclosed in these documents.

Citation List

PTL

PTL 1: WO2008/047863 (also published as EP 2 087 890 A1)

PTL 2: WO2004/032965

PTL 3: JP2004-115413A

PTL 4: Japanese Patent No. 4459543

PTL 5: Japanese Patent No. 4685090

PTL 6: JP2008-137975A

PTL 7: JPH07-41432A

PTL 8: JP2008-137975A

PTL 9: JP2011-516443A

PTL 10: JP2004-91450A

PTL 11: JP2005-206491A

NPL

NPL 1: Am J Respir Crit Care Med 177, pp.195-201, 2008
NPL 2: Am J Physiol Gastrointest Liver Physiol 302: G420-G429, 2012
NPL 3: Cell, 43 (10), pp.26-35, 2011
NPL 4: Cell, 44 (2), pp.34-40, 2012
NPL 5: Biochem. Biophys. Res. Comm, 122, pp.145-1459, 1984
NPL 6: Development, 125, pp.1315-1324, 1998
NPL 7: Am J Respir Crit Care Med, 162, pp.707-715, 2000
NPL 8: Circulation, 110, pp.2896-2902, 2004
NPL 9: Am J Respir Cell Mol Biol, 26, pp.525-533, 2002
NPL 10: Circulation, 111, pp.1407-1414, 2005
NPL 11: Am J Respir Cell Med Bio, 32, 268-280, 2005
NPL 12: Nature Medicine, 5, pp.226-230, 1999
NPL 13: Am J Respir Crit Care Med, 156, pp.1937-1944, 1997
NPL 14: Am J Respir Crit Care Med, 172, pp.1575-1580, 2005
NPL 15: Circ J, 77, 2127-2133, 2013
NPL 16: Am J Respir Crit Care Med, 177, pp.195-201, 2008
NPL 17: Am J Physiol Lung Cell Mol Physiol, 290, pp.L59-L65, 2006

NPL 18: European Respiratory Society (ERS 2010), Barcelona 2010, Annual Congress, Spain
NPL 19: Clinical & Experimental Allergy, 40, pp.317-326, 2009
NPL 20: Am J Respir Cell Mol Biol, 47 (2), pp.170-177, 2012
NPL 21: The 60th Fall Meeting of the Japanese Society of Allergology, 2010
NPL 22: American Thoracic Society 2011 International Conference, Denver, Colorado
NPL 23: Arch Biochem Biophys, 212, 196, 1981
NPL 24: Adv Drug Derliv Rev, 31, 287-301, 1998
NPL 25: JR Soc Interface, 6 Suppl. 3, S311-S324, 2009
NPL 26: J Biomater Sci Polym Edn, 12, 77-88, 2001
NPL 27: Drug Deliv Syst, 23 (3), p.394, 2008
NPL 28: General Thoracic and Cardiovascular Surgery Vol. 51, p.281, 2003

## Summary of Invention

### Technical Problem

As described above, the present inventors developed a microsphere (MS) preparation of a low molecular weight compound and have already reported on the clinical application of the MS preparation by subcutaneous administration or intramuscular administration. However, in addition, the development of a lung-specific disease therapeutic agent has been desired. Accordingly, an object of the present invention is to provide a lung-specific disease therapeutic agent that can be intravenously administered.

### Solution to Problem

The present inventors found that intravenous administration of a MS preparation results in lung-tissue-specific accumulation and the medicine shows an specific effect for a lung disease by being gradually released the medicine in the lungs. As a result that a further study has been repeated, the inventors succeeded in developing a lung disease-specific therapeutic agent.
The present invention includes the following items 1 to 11.
1. A lung-specific therapeutic agent for use in the therapy of a pulmonary disease, wherein the therapeutic agent comprises sustained-release microspheres containing a pharmaceutical compound, wherein the microspheres have a number average particle size of 20 to 40 μm, and a maximum particle size of 100 μm or less, wherein the therapeutic agent is to be intravenously administered, wherein the pharmaceutical compound is a pulmonary disease therapeutic drug.

2. The lung-specific therapeutic agent for use according to item 1, wherein the pulmonary disease therapeutic drug is at least one member selected from the group consisting of beclomethasone, fluticasone, montelukast, tiotropium, imidafenacin, beraprost, carbacyclin, sivelestat, tulobuterol, salmeterol, and salts thereof.

3. The lung-specific therapeutic agent for use according to item 1, wherein the pulmonary disease therapeutic drug is at least one in vivo regeneration factor selected from the group consisting of b-FGF, HGF, EGF, SDF-1, IGF-1, LIF, HMGB1, G-CSF, and extracellular matrices.

4. The lung-specific therapeutic agent for use according to item 1, wherein the pulmonary disease therapeutic drug is a compound represented by Formula (I)

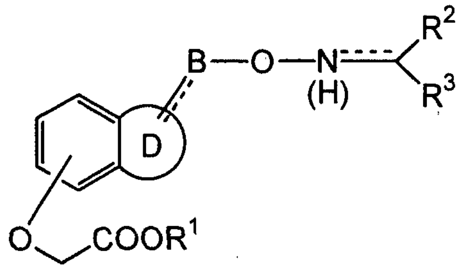

(I)

wherein

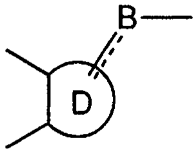

represents

(i) 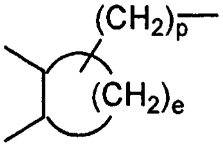 , (ii) 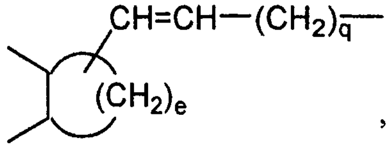 ,

(iii) 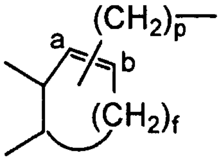 or (iv) 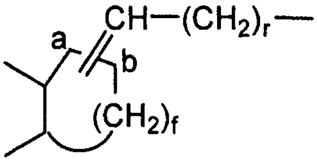 ,

wherein $R^1$ represents hydrogen or $C_{1-4}$ alkyl,
$R^2$ represents (i) hydrogen, (ii) $C_{1-8}$ alkyl, (iii) phenyl or $C_{4-7}$ cycloalkyl, (iv) a 4- to 7-membered monocycle containing one nitrogen atom, (v) a benzene ring- or $C_{4-7}$ cycloalkyl-substituted $C_{1-4}$ alkyl group, or (vi) a $C_{1-4}$ alkyl group substituted with a 4- to 7-membered monocycle containing one nitrogen atom,
$R^3$ represents (i) $C_{1-8}$ alkyl, (ii) phenyl or $C_{4-7}$ cycloalkyl, (iii) a 4- to 7-membered monocycle containing one nitrogen atom, (iv) a benzene ring- or $C_{4-7}$ cycloalkyl-substituted $C_{1-4}$ alkyl group, or (v) a $C_{1-4}$ alkyl group substituted with a 4- to 7-membered monocycle containing one nitrogen atom,
e represents an integer of 3 to 5,
f represents an integer of 1 to 3,
p represents an integer of 1 to 4,
q represents 1 or 2, and
r represents an integer of 1 to 3;

provided that when

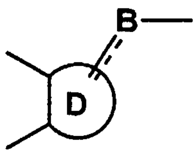

is a group defined in (iii) or (iv), each of $-(CH_2)_p-$ and $=CH-(CH_2)_s-$ bonds to either position a or b on the ring, and the rings in $R^2$ and $R^3$ may be substituted with one to three substituents selected from the group consisting of $C_{1-4}$ alkyl groups, $C_{1-4}$ alkoxy groups, halogen atoms, nitro groups, and trihalomethyl groups, or a salt thereof.

The lung-specific therapeutic agent for use according to item 4, wherein the pulmonary disease therapeutic drug is (E)-[5-[2-[1-phenyl-1-(3-pyridyl)methylideneaminooxy]ethyl]-7,8-dihydronaphthalen-1-yloxy]acetic acid.

The lung-specific therapeutic agent for use according to item 1, wherein the pulmonary disease therapeutic drug is at least one member selected from the group consisting of (±)-(1R,2R,3aS,8bS)-2,3,3a,8b-tetrahydro-2-hydroxy-1-[(E)-(3S,4RS)-3-hydroxy-4-methyl-1-octen-6-iny1]-1H-cyclopenta[b]benzofuran-5-butanoic acid (beraprost), 2-{4-[N-(5,6-diphenylpyrazin-2-yl)-N-isopropylamino]butyloxy} acetic acid (MRE-269), carbacyclin, and salts thereof.

The lung-specific therapeutic agent for use according to item 1, wherein the sustained-release microspheres contain a biodegradable polymer selected from the group consisting of gelatin hydrogels, liposomes, lipids, polylactic acids, lactic acid-glycolic acid copolymers, polyglycolic acids, polydioxanes, and mixtures thereof.

The lung-specific therapeutic agent for use according to item 1, which is a sustained-release microsphere preparation comprising a pulmonary disease therapeutic drug selected from the group consisting of beclomethasone, fluticasone, montelukast, tiotropium, imidafenacin, beraprost, carbacyclin, sivelestat, tulobuterol, salmeterol, and salts thereof; and at least one biodegradable polymer selected from the group consisting of gelatin hydrogels, liposomes, lipids, polylactic acids, lactic acid-glycolic acid copolymers, polyglycolic acids, polydioxanes, and mixtures thereof.

The lung-specific therapeutic agent for use according to item 1, which comprises said sustained-release microsphere preparation containing at least one pulmonary disease therapeutic drug selected from the group consisting of (E)-[5-[2-[1-phenyl-1-(3-pyridyl)methylideneaminooxy]ethyl]-7,8-dihydronaphthalene-1-yloxy]acetic acid, (±)-(1R,2R,3aS,8bS)-2,3,3a,8b-tetrahydro-2-hydroxy-1-[(E)-(3S,4RS)-3-hydroxy-4-methyl-1-octene 6-inyl]-1H-cyclopenta[b]benzofuran-5-butanoic acid (beraprost), 2-{4-[N-(5,6-diphenylpyrazin-2-yl)-N-isopropylamino]butyloxy}acetic acid (MRE-269), and salts thereof; and a biodegradable polymer selected from the group consisting of polylactic acids, lactic acid-glycolic acid copolymers, and mixtures thereof.

The lung-specific therapeutic agent for use according to item 1, which comprises sustained-release microspheres containing at least one pulmonary disease therapeutic drug selected from the group consisting of
beclomethasone, fluticasone, montelukast, tiotropium, imidafenacin, beraprost, carbacyclin, sivelestat, tulobuterol, salmeterol;
(E)-[5-[2-[1-phenyl-1-(3-pyridyl)methylideneaminooxy]ethyl]-7,8-dihydronaphthalen-1-yloxy]acetic acid;
(±)-(1R,2R,3aS,8bS)-2,3,3a,8b-tetrahydro-2-hydroxy-1-[(E)-(3S,4RS)-3-hydroxy-4-methyl-1-octene6-inyl]-1H-cyclopenta[b]benzofuran-5-butanoic acid (beraprost);
2-{4-[N-(5,6-diphenylpyrazin-2-yl)-N-isopropylamino]butyloxy}acetic acid (MRE-269); and
salts thereof,
the therapeutic agent is to be administered at a dose of 6 mg/kg or less in terms of the sustained-release microspheres.

The lung-specific therapeutic agent for use according to any one of items 1 to 10, wherein the pulmonary disease is at least one member selected from the group consisting of acute pneumonia, fibroid lung, interstitial pneumonia, pulmonary hypertension, chronic obstructive pulmonary diseases (COPD), chronic bronchitis, pulmonary emphysema, asthma, intractable asthma, systemic inflammatory response syndrome (SIRS), acute lung injury (ALI), respiratory distress syndrome (ARDS), sarcoidosis, chronic idiopathic pulmonary thromboembolism, diffuse panbronchiolitis, cystic fibrosis, hypersensitivity pneumonitis, lung cancer, obesity hypoventilation syndrome, alveolar hypoventilation syndrome, and chronic rejection in lung transplantation.

## Advantageous Effects of Invention

The present invention provides a lung-specific disease therapeutic agent that is to be intravenously administered.

## Brief Description of Drawings

Fig. 1 is a graph of an in vitro release test using the preparation of Preparation Example 1.
Fig. 2 is a graph of an in vitro release test using the preparation of Preparation Example 3.
Fig. 3 is a graph of an in vitro release test using the preparation of Preparation Example 4.
Fig. 4 is a graph of a blood kinetics test by in vivo subcutaneous administration of the preparation of Preparation Example 1 to rats.
Fig. 5 is a graph of survival rate curves after intravenous administration of Preparation Example 2 (PLGA-MS (negative control)) to pulmonary hypertension models.
Fig. 6 is a graph of the survival benefit from intravenous administration, of Preparation Example 1 (Compound 1-MS) .
Fig. 7 is a graph of pulmonary artery pressure (right ventricular pressure).
Fig. 8 is a graph of the ratio of right ventricular pressure to left ventricular pressure.
Fig. 9 is a graph of the ratio of right ventricular weight to left ventricular plus septal weight.
Fig. 10 is a graph showing the effect enhancement achieved by administering a $TXA_2$ synthetase inhibitor ozagrel in combination with Compound 3 or 7.

## Description of Embodiments

Features of the lung-specific therapeutic agent of the present invention are that the therapeutic agent comprises sustained-release microspheres containing a pharmaceutical compound and is intravenously administered.

Sustained-Release Microspheres

The lung-specific therapeutic agent of the present invention contains sustained-release microspheres.

Generally, microspheres (or microcapsules) refer to a dosage form of small spherical particles (with a particle size of 1 to 999 $\mu$m) comprising a pharmaceutical compound encapsulated in a shell formed of a polymer.

In this specification, the term "lung-specific" means selective transfer to the lungs. Selective transfer to the lung is considered to be based on the mechanism that the lung-specific therapeutic agent of the present invention is selectively trapped in the lungs. However, the present invention is not limited to this mechanism. Selective transfer to the lungs can be directly confirmed, for example, by measuring the concentration in the lungs of the lung-specific therapeutic agent of the present invention or a pharmaceutical compound contained in the agent, or it can be indirectly confirmed by enhanced efficacy achieved by selecting the dosage form of the present invention.

Pharmaceutical Compound

The "pharmaceutical compound" of the present invention is a pulmonary disease therapeutic drug such as antithrombotic agents, circulation-improving agents, smooth muscle dilators, analgesics or anti-inflammatory agents, local anesthetics, metabolism improvers, elastase inhibitors, prostaglandins, in vivo regeneration factors, and in vivo regeneration factor inducers. The pharmaceutical compound used in the present invention includes not only those found so far but also those that will be found in the future.

The pharmaceutical compound used in the present invention may be, for example, any of various substances such as low molecular weight compounds, proteins or polypeptides, or glycoproteins (e.g., extracellular matrices and cell adhesion factors), polynucleotides (e.g., sense nucleotides, antisense nucleotides, and decoy nucleotides), vaccines, and antibodies.

The pharmaceutical compound used in the present invention may be in various forms, such as free forms, salts, solvates (e.g., hydrates), and complexes.

The pharmaceutical compound used in the present invention is a pulmonary disease therapeutic drug.

The pulmonary disease therapeutic drug may be at least one member selected from the group consisting of steroid drugs (e.g., beclomethasone and fluticasone), leukotriene receptor antagonists (LTRA) (e.g., montelukast), M3 receptor antagonists (e.g., tiotropium and imidafenacin), $PGI_2$ agonists (e.g., beraprost and carbacyclin), $EP_2$ agonists, $EP_4$ agonists, elastase inhibitors (e.g., sivelestat), $\beta$2 adrenoreceptor agonists (e.g., tulobuterol and salmeterol), in vivo regeneration factors, and in vivo regeneration factor inducers.

Examples of pulmonary diseases include acute pneumonia, pulmonary fibrosis, interstitial pneumonia (e.g., acute interstitial pneumonia, diffuse interstitial pneumonia, and lymphoid interstitial pneumonia), pulmonary hypertension, chronic obstructive pulmonary disease (COPD), chronic bronchitis, pulmonary emphysema, asthma, intractable asthma, systemic inflammatory response syndrome (SIRS), acute lung injury (ALI), acute respiratory distress syndrome (ARDS), sarcoidosis (e.g., pulmonary sarcoidosis, ocular sarcoidosis, cardiac sarcoidosis, and cutaneous sarcoidosis), chronic idiopathic pulmonary thromboembolism, diffuse panbronchiolitis, cystic fibrosis, hypersensitivity pneumonia, obesity hypoventilation syndrome, alveolar hypoventilation syndrome, lung cancer, and chronic rejection in lung transplantation. The present invention is suitable for application to at least one disease selected from pulmonary fibrosis, interstitial pneumonia, pulmonary hypertension, asthma, COPD, SIRS, and the like.

The pulmonary disease therapeutic drug is preferably, for example, at least one agent selected from the group consisting of steroids, $\beta$2 agonists, LT antagonists, M3 antagonist, prostaglandins, elastase inhibitors, in vivo regeneration factors, and in vivo regeneration factor production inducers.

The pulmonary disease therapeutic drug can be preferably, for example, at least one member selected from the group consisting of beclomethasone, fluticasone, montelukast, tiotropium, imidafenacin, beraprost, carbacyclin, sivelestat, tulobuterol, salmeterol, and salts thereof.

Examples of the antithrombotic agents include heparin preparations (e.g., heparin sodium, heparin calcium, and dalteparin sodium), oral anticoagulants (e.g., warfarin potassium), antithrombin agents (e.g., gabexate mesylate, nafamostat mesylate, and argatroban), anti-platelet aggregation inhibitors (e.g., aspirin, dipyridamole, ticlopidine hydrochloride, beraprost sodium, cilostazol, ozagrel sodium, ozagrel hydrochloride, sarpogrelate hydrochloride, and ethyl eicosapentanaate), thrombolytic agents (e.g., urokinase, tisokinase, alteplase, nateplase, monteplase, and pamiteplase), factor Xa inhibitors, and factor VIIa inhibitors.

Examples of the circulation improving agents include ifenprodil tartarate, aniracetam, donepezil hydrochloride, amantadine hydrochloride, nicergoline, ibudilast, papaverine compounds, nicotine compounds, calcium antagonists (e.g., nifedipine, amlodipine, diltiazem, and azelnidipine), $\beta$-receptor agonists (e.g., ephedrine, salbutamol, procaterol, salmeterol, and mabuterol), $\alpha$-receptor antagonists (e.g., uradipil, terazosin, doxazosin, bunazosin, and prazosin), angiotensin II receptor antagonists; ARB (e.g., losartan, candesartan, valsartan, and telmisartan), and PDE inhibitors (e.g., theophylline, milrinone, tadalafil, dipyridamole, and sildenafil).

Examples of the analgesics or anti-inflammatory agents include non-steroidal anti-inflammatory drugs (NSAID) such as aspirin, indomethacin, diclofenac, meloxicam, and celecoxib; opioid analgesics such as codeine and morphine; and pentazocine, buprenorphine hydrochloride, and eptazocine hydrobromide.

Examples of the local anesthetics include steroids, procaine, cocaine hydrochloride, lidocaine hydrochloride, and ropivacaine hydrochloride.

Examples of the metabolism improvers include anti-hyperlipidemic agents such as HMG-CoA reductase inhibitors (e.g., atorvastatin, simvastatin, and pravastatin); and antidiabetic drugs such as PPAR-y agonists (e.g., thiazolidine derivatives such as pioglitazone and rosiglitazone, adiponectin, and leptin), DPP-IV inhibitors (e.g., sitagliptin, vildagliptin, and alogliptin), and GLP-1 agonists.

Examples of the elastase inhibitors include sivelestat. Examples of prostaglandins include $PGE_1$, $PGE_2$, $PGI_2$, and derivatives thereof, lipo$PGE_1$, 6-oxy-$PGE_1$, 6-oxy-$PGE_1$ derivatives, ornoprostil, limaprost, enprostil, and misoprostol.

Examples of the in vivo regeneration factors include vascular endothelial growth factor (VEGF), hepatocyte growth factor (HGF), various fibroblast growth factors (a/b FGF), transformation growth factor-$\beta$ (TGF-$\beta$), platelet-derived growth factor (PDGF), angiopoietin, hypoxia-inducible factor (HIF), insulin-like growth factor (IGF), bone morphogenetic protein (BMP), connective tissue growth factor (CTGF), epidermal growth factor (EGF), nerve growth factor (NGF), brain-derived neurotrophic factor (BDNF), glia-cell-derived neurotrophic factor (GDNF), stem cell factor (SCF), stromal cell-derived factor (SDF-1), granulocyte colony-stimulating factor (G-CSF), keratinocyte growth factor (KGF), chondrocyte growth factor (GDF), leukemia inhibitory factor (LIF), HMGB1, and Kruppel-like transcription factors (KLF), and growth factors of their families.

Examples of the in vivo regeneration factors further include extracellular matrices (e.g., fibronectins, laminins, and proteoglycans) and cell adhesion factors (e.g., cadherins and integrins).

The in vivo regeneration factor can be preferably, for example, at least one member selected from the group consisting of b-FGF, HGF, EGF, SDF-1, HMGB1, IGF-1, LIF, G-CSF, and extracellular matrices.

The in vivo regeneration factor inducer is an agent for inducing the production of an in vivo regeneration factor. Examples include $PGI_2$ agonists, $EP_2$ agonists, $EP_4$ agonists, cholera toxin, 8-bromo-cAMP, dibutyryl-cAMP, forskolin, AT1 receptor antagonist (ARB), peroxisome proliferator-activated receptor gamma (PPAR-$\gamma$) agonist, phosphodiesterase (PDE) inhibitors, IL-1, TNF-$\alpha$, and INF.

Examples of pharmaceutical compounds of the pulmonary disease therapeutic agent of the present invention and other combination drugs for complementing and/or enhancing preventive and/or therapeutic effects include not only those found so far but also those that will be found in the future, based on the mechanism mentioned above.

Examples of preferable in vivo regeneration factor inducers include at least one member selected from the group consisting of $PGI_2$ agonists, $EP_2$ agonists, $EP_4$ agonists, AT1 receptor antagonists (angiotensin II type 1 antagonists; ARB), peroxisome proliferator-activated receptor gamma (PPAR-$\gamma$) agonists, and phosphodiesterase (PDE) inhibitors. More preferably, the in vivo regeneration factor inducer may be, for example, at least one member selected from the group consisting of $PGI_2$ agonists, $EP_2$ agonists, and $EP_4$ agonists. More preferably, the in vivo regeneration factor inducer may be a $PGI_2$ agonist.

Examples of $PGI_2$ agonists that can be used in the present invention include $PGE_1$ and $PGI_2$, derivatives thereof (e.g., 6-oxy-$PGE_1$, ornoprostil, limaprost, enprostil, and misoprostol), prodrugs thereof, depot preparations persistent preparations (sustained-release preparations) thereof (e.g., lipo$PGE_1$). Examples of $EP_2$ and $EP_4$ agonists include various PGE derivatives, prodrugs thereof, and depot formulations (sustained-release preparations) thereof.

The $EP_2$ agonist as referred to herein includes all of the $EP_2$ agonists so far known and $EP_2$ agonists that will be discovered in the future. Examples of preferable $EP_2$ agonists include compounds described in EP 0 860 430 A, U.S. Patent No. 6,110,969, WO99/33794, EP 974 580 A, WO95/19964, WO98/28264, WO99/19300, EP 0 911 321 A, WO98/58911, U.S. Patent No. 5,698,598, U.S. Patent No. 6,376,533, U.S. Patent No. 4,132,738, or U.S. Patent No. 3,965,143. Particularly preferable $EP_2$ agonists are (5Z,9$\beta$,11$\alpha$,13E)-17,17-propano-11,16-dihydroxy-9-chloro-20-norprost-5,13-dienoic acid and salts thereof.

The $EP_4$ agonist as referred to herein includes all of the $EP_4$ agonists so far known and $EP_4$ agonists that will be discovered in the future. Examples of preferable $EP_4$ agonists include compounds disclosed in WO00/03980, WO99/02164, WO00/16760, WO00/18744, WO00/21542, WO00/38663, WO00/38690, WO00/38667, WO00/40248, WO00/54808, WO00/54809, WO01/10426, EP 1 110 949 A, EP 1 121 939 A, EP 1 132 086 A, WO2001/72268, JP2002-104939A, WO02/42268, JP2002-179595A, WO02/47669, WO02/64564, WO03/035064, WO03053923, and U.S. Patent No. 6,552,067. Examples of particularly preferable $EP_4$ agonists include (11$\alpha$, 13E, 15$\alpha$)-9-oxo-11,15-dihydroxy-16-{3-methoxymethylphenyl)-17,18,19,20-tetranor-5-thiaprost-13-enoic acid, and esters thereof.

When the in vivo regeneration factor is a protein, the in vivo regeneration factor inducer may be a gene encoding the protein.

The $PGI_2$ agonist (IP receptor agonist) as referred to herein includes all of the $PGI_2$ agonists so far known and $PGI_2$ agonists that will be discovered in the future.

For example, the $PGI_2$ agonist is preferably a compound represented by Formula (I), or a salt thereof. Formula (I):

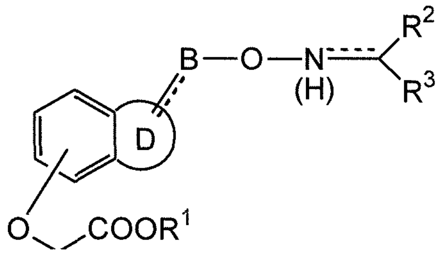

(I)

In Formula (I),

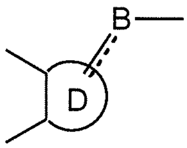

is preferably

(i) 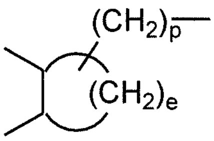 ,

(ii) 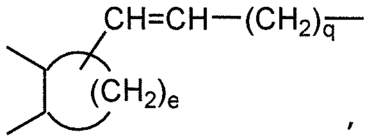 ,

(iii) 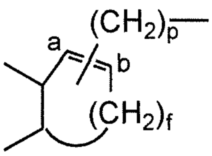 or (iv) 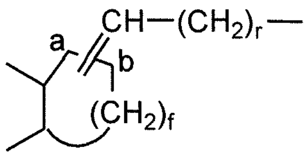 ,

and more preferably

(iii) 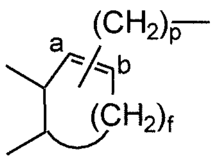 .

In Formula (I), $R^2$ is preferably

(iii) phenyl or $C_{4-7}$ cycloalkyl,
(iv) a 4- to 7-membered monocycle containing one nitrogen atom,
(v) a benzene ring- or $C_{4-7}$ cycloalkyl-substituted $C_{1-4}$ alkyl group, or
(vi) a $C_{1-4}$ alkyl group substituted with a 4- to 7-membered monocycle containing one nitrogen atom.

$R^2$ is more preferably

(iii) phenyl or $C_{4-7}$ cycloalkyl, or
(iv) a 4- to 7-membered monocycle containing one nitrogen atom.

In Formula (I), $R^3$ is preferably

(ii) phenyl or $C_{4-7}$ cycloalkyl,
(iii) a 4- to 7-membered monocycle containing one nitrogen atom,
(iv) a benzene ring- or $C_{4-7}$ cycloalkyl-substituted $C_{1-4}$ alkyl,
(v) a $C_{1-4}$ alkyl group substituted with a 4- to 7-membered monocycle containing one nitrogen atom.

$R^3$ is more preferably

(ii) phenyl or $C_{4-7}$ cycloalkyl, or
(iii) a 4- to 7-membered monocycle containing one nitrogen atom.

The compound represented by Formula (I) or a salt thereof is preferably one of the following compounds that are oxime derivatives.

Compound 1: (E)-[5-[2-[1-phenyl-1-(3-pyridyl)methylideneaminooxy]ethyl]-7,8-dihydronaphthalen-1-yloxy]acetic acid

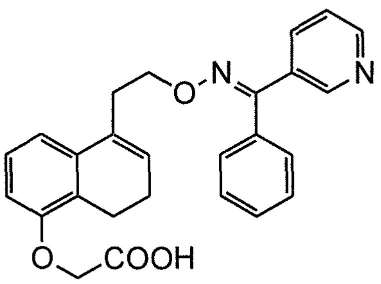

Compound 2: (Z)-[5-[2-[1-phenyl-1-(3-pyridyl)methylideneaminooxy]ethyl]-7,8-dihydronaphthalen-1-yloxy]acetic acid

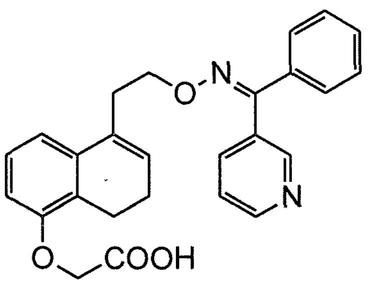

Examples of other PGI2 agonists include beraprost sodium (Compound 3): (sodium (±)-(1R,2R,3aS,8bS)-2,3,3a,8b-tetrahydro-2-hydroxy-1-[(E)-(3S,4RS)-3-hydroxy-4-methyl-1-octen-6-ynyl]-1H-cyclopenta[b]benzofuran-5-butanoate), OP-2507 (Compound 4): (5-{(3aR,4R,6aS)-5-hydroxy-4-[(1E,3S)-3-hydroxy-3-(cis-4-propylcyclohexyl)prop-1-enyl-3,3a,4,5,6,6a-hexahydrocyclopenta[b]pyrrol-2-yl}pentanoic acid methyl ester), MRE-269 (Compound 5): 2-{4-[N-(5,6-diphenylpyrazin-2-yl)-N-isopropylamino]butoxy}acetic acid,
Compound 6 (NS-304) that is a derivative of Compound 5: 2-{4-[(5,6-diphenylpyrazin-2-yl)-N-isopropylamino]butoxy}-N-(methylsulfonyl)acetamide,
ornoprostil (Compound 7) that is a $PGE_1$ derivative: 17S,20-dimethyl-6-oxo-prostaglandin E1 methyl ester,
limaprost that is a $PGE_1$ derivative: (E)-7-[(1R,2R,3R)-3-hydroxy-2-[(3S,5S)-E-3-hydroxy-5-methyl-1-nonenyl]-5-oxocyclopentyl]-2-heptanoic acid, and carbacyclin derivatives that are $PGI_2$ derivatives.

Among the derivatives represented by Formula (I) and salts thereof, Compound 1, 3, or 5, which have chemical stability, is preferably used to produce microsphere (MS) preparations.

The present inventors have now found that addition of Compound 1 to fibroblasts promotes the production of HMGB1 (high-mobility group B1) (see Pharmacological Test 6 below). Based on the cytokine production promoting action mentioned above, Compound 1 newly produces bone-marrow-cell-mobilizing factors (e.g., HMGB1) from fibroblasts, etc., and the factors are released into blood to supply marrow bone marrow mesenchymal stem cells (MSC), vascular endothelial progenitor cells (EPC), etc., from bone marrow to blood, and the supplied MSC is accumulated at

the site of injury by bone-marrow cell-accumulation factors (e.g. SDF-1). It was suggested that the accumulated MSC produces blood vessel/tissue regeneration effects with effector factors that differentiate and proliferate cells necessary for each tissue regeneration. For example, it was confirmed that the anti-heart-failure effect of Compound 1 was attenuated by a SDF-1 receptor (CXCR4) antagonist (PLoS ONE 8(7): e69302, 2013), an anti-HGF neutralizing antibody (Biomedicine & Aging Pathology 1: 90-96, 2011), an anti-VEGF neutralizing antibody (Clinical Science 112; 607-616, 2007), etc.

Further, Compound 1, which also has a $TXA_2$ synthase inhibitory effect, was confirmed to promote endogenous $PGI_2$ and $PGE_2$ production (described in Pharmacological Test 6 below).

The results thus confirmed that Compound 1, which also has a $TXA_2$ synthase inhibitory effect, acts not only as an IP receptor agonist but also has $PGE_2$ actions ($EP_2$ and $EP_4$ receptor agonists). As another pharmacological effect of Compound 1, HMGB1 and endogenous $PGE_2$ production-promoting effects have been newly found. This revealed the potential efficacy of Compound 1 for new target diseases.

Such new diseases include, for example, specific diseases designated as target intractable diseases in the clinical research field of the Research Project on Measures for Intractable Diseases funded by the Japanese Ministry of Health, Labor and Welfare (about 130 diseases in 2012).

Of these specific diseases, Compound 1 is expected to be effective, for example, for the following diseases: Behcet's disease, spinal muscular atrophy, multiple sclerosis, bulbospinal muscular atrophy, myasthenia gravis, chronic inflammatory demyelinating polyneuropathy, systemic lupus erythematosus, hypertrophic cardiomyopathy, subacute myelo-optico-neuropathy, restrictive cardiomyopathy, aplastic anemia, mitochondrial diseases, lymphangioleiomyomatosis (LAM), severe erythema exsudativum multiforme (acute phase), scleroderma/dermatomyositis, polymyositis, ossification of the ligamentum flavum, idiopathic thrombocytopenic purpura, diencephalohypophysial dysfunction, periarteritis nodosa, inappropriate prolactin (PRL) secretion syndrome, polyarteritis nodosa, inappropriate gonadotropin secretion syndrome, microscopic polyangiitis, inappropriate ADH secretion syndrome, ulcerative colitis, aortitis syndrome, Cushing's disease, Buerger's disease (Buerger disease), acromegaly, pemphigus, hypopituitarism, spinocerebellar degeneration, Crohn's disease, fulminant hepatitis, malignant rheumatoid arthritis, Parkinson's disease and related diseases, progressive supranuclear palsy, corticobasal degeneration, amyloidosis, ossification of the posterior longitudinal ligament, Huntington's disease, moyamoya disease (spontaneous occlusion of circle of Willis), idiopathic dilated (congestive) cardiomyopathy, multiple system atrophy, striatonigral degeneration, olivopontocerebellar atrophy, Shy-Drager syndrome, epidermolysis bullosa (junctional epidermolysis bullosa and dystrophic epidermolysis bullosa), pustular psoriasis, extensive vertebral canal stenosis, primary biliary cirrhosis, severe acute pancreatitis, idiopathic osteonecrosis of femoral head, mixed connective tissue disease, primary immunodeficiency syndrome, idiopathic interstitial pneumonia, retinitis pigmentosa, prion disease, Creutzfeldt-Jakob disease, Gerstmann-Straussler-Scheinker disease, pulmonary arterial hypertension, neurofibromatosis type I/neurofibromatosis type II, subacute sclerosing panencephalitis, Budd-Chiari syndrome, chronic thromboembolic pulmonary hypertension, lysosomal disease, Fabry disease, and adrenoleukodystrophy.

U.S. Patent No. 5,480,998 discloses that a Compound 1 that is an oxime (OX) derivative represented by Formula (1) and used as a $PGI_2$ agonist (IP agonist) in the present invention, or a nontoxic salt thereof has the activities of platelet aggregation inhibition, platelet adhesion inhibition, vasodilation, and gastric acid secretion inhibition; therefore, Compound 1 or a non-toxic salt thereof is useful for preventing and/or treating thrombosis, arteriosclerosis, ischemic heart disease, gastric ulcer, hypertension, and the like.

Further, WO2004/032965 and WO2008/047863 disclose neovascularization action, differentiation-inducing action in various stem cells, anti-apoptosis action, anti-fibrosis action, etc., based on induction of in vivo regeneration factor production for repair of various cell or organ dysfunctions.

Methods for Producing the Compounds Represented by Formula (I)

Among the $PGI_2$ agonists that can be used in the present invention, for example, the compounds represented by Formula (I) can be produced by using the method disclosed in U.S. Patent No. 5,480,998.

For example, (E)-[5-[2-[1-phenyl-1-(3-pyridyl)methylideneaminooxy]ethyl]-7,8-dihydronaphthalen-1-yloxy]acetic acid (Compound 1) is disclosed in Example 2(g) of U.S. Patent No. 5,480,998.

A method for producing sodium (±)-{(1R,2R,3aS,8bS)-2,3,3a,8b-tetrahydro-2-hydroxy-1-[(1E,3S)-3-hydroxy-4-methyl-1-octen-6-yn-1-yl]-1H-benzo[b]cyclopenta[d]furan-5-yl}butanoate (beraprost sodium; Compound 3) is disclosed in WO1996/026721.

A method for producing 2-{4-[N-(5,6-Diphenylpyrazin-2-yl)-N-isopropylamino]butyloxy}acetic acid (MRE-269; Compound 5) and a derivative thereof (Compound 6) is disclosed in WO02/088084.

In the present invention, various Compound 1-containing in vivo regeneration factor inducers, in vivo regeneration factor proteins and peptides comprising the active sites of the in vivo regeneration factor proteins, genes thereof, and various pharmaceutical compounds may be used singly or in a combination of two or more. These may be in the form of microsphere preparations comprising biodegradable polymers described below, such as various lactic acid-glycolic acid copolymers, lactic acid polymers, gelatin hydrogels, and liposomes. These may be used singly or in a combination of two or more.

Polymers

Sustained-release microspheres that are used in the present invention preferably comprise a biodegradable polymer as a polymer for forming a shell. Based on this feature, the sustained-release microspheres used in the present invention have the property of releasing a pharmaceutical compound in a sustained manner.

The mechanism for controlling the rate of sustained release from a biodegradable polymer may be, for example, a degradation control mechanism, a diffusion control mechanism, or a membrane permeation control mechanism.

The biodegradable polymer may be a natural polymer or a synthetic polymer.

Examples of the biodegradable polymer include natural polymers or synthetic polymers, such as fatty acid ester polymers or copolymers thereof, polyacrylic acid esters, polyhydroxybutyric acids, polyalkylene oxalates, polyorthoesters, polycarbonate, and polyamino acids.

Examples of the fatty acid ester polymers or copolymers thereof include polylactic acid (PLA), polyglycolic acid (PGA), polydioxane (PDS), polycitric acid, polymalic acid, polyethylene succinate, polybutylene succinate, poly-s-caprolactone, polybutylene terephthalate adipate, gelatin, gelatin hydrogel, collagen, atelocollagen, fibrin, hyaluronic acid or lactic acid-glycolic acid copolymers (PLGA), poly-$\alpha$-cyanoacrylic acid ester, poly-p-hydroxybutyric acid, polytrimethylene oxate, polyorthoester, polyorthocarbonate, polyethylene carbonate, poly-$\gamma$-benzyl-L-glutamic acid, polyvinyl alcohol, polyester carbonate, polyacid anhydride, polycyanoacrylate, polyphosphazene, liposomes, and poly-L-alanine.

The lactic acid-glycolic acid copolymer preferably has a compositional ratio of lactic acid to glycolic acid in the range of about 100/0 to about 50/50 (w/w).

Such biodegradable polymers may be used singly or in a combination of two or more.

The biodegradable polymer is preferably at least one member selected from the group consisting of gelatin hydrogels, liposomes, lipids, polylactic acids (PLA), a lactic acid-glycolic acid copolymer, polyglycolic acids (PGA), polyglycolic acids or lactic acid-glycolic acid copolymers (PLGA), and polydioxane. The biodegradable polymer is more preferably at least one member selected from the group consisting of gelatin hydrogels, polylactic acids, polyglycolic acids, and lactic acid-glycolic acid copolymers (PLGA). The biodegradable polymer is even more preferably at least one member selected from the group consisting of gelatin hydrogels, polyglycolic acids, and lactic acid-glycolic acid copolymers (PLGA).

From the viewpoint of appropriate sustained release, the sustained-release microsphere preparation preferably comprises a biodegradable polymer selected from the group consisting of gelatin hydrogels, liposomes, lipids, polylactic acids, lactic acid-glycolic acid copolymers, polyglycolic acids, polydioxane, and mixtures thereof.

The biodegradable polymer used in the present invention preferably has a weight average molecular weight of 1,000 to 50,000, although it may vary according to the type of biodegradable polymer, etc.

More specifically, the lactic acid-glycolic acid copolymer preferably has a weight average molecular weight of about 1,000 to 800,000, and more preferably about 1,000 to 100,000.

The polylactic acid preferably has a weight average molecular weight of about 1,000 to 100,000, and more preferably about 1,000 to 50,000.

The lactic acid-glycolic acid copolymer preferably has a weight average molecular weight of about 1,000 to 100,000, and more preferably about 1,000 to 50,000.

In this specification, the "weight average molecular weight" refers to a value in terms of polystyrene as measured by gel permeation chromatography (GPC).

These biodegradable polymers can be synthesized in accordance with known production methods.

In order to control initial burst, etc., sustained-release microspheres used in the present invention may comprise chitosan, dibutylhydroxytoluene (BHT), tocopherol (vitamin E), or amino acids (e.g., alginic acid).

Erythrocytes have a diameter of 7 to 8 $\mu$m and a thickness of about 2 $\mu$m. If the microsphere (MS) preparation has a number average particle size of about 10 $\mu$m or less, which is equivalent to or smaller than the size of erythrocytes, efficient long-term trapping of a microsphere (MS) preparation in the lungs is unlikely to occur. Accordingly, to trap the MS preparation of the present invention in the lungs with high efficiency, the MS preparation preferably has a number average particle size of 10 $\mu$m or larger, and more preferably 20 $\mu$m or larger.

According to the calculation of Weibel, normal human lungs have 100,000 to 200,000 small arteries with a diameter of 300 to 500 $\mu$m, and each small artery is considered to include 100 twigs thereof with a diameter of 50 to 300 $\mu$m, 2,000 arterioles with a diameter of 10 to 50 $\mu$m, and 200,000 capillaries with a diameter of 10 $\mu$m or less (Nuclear medicine 16: 927-931, 1979). Accordingly, in order to avoid pulmonary embolism with high efficiency, the microsphere (MS) preparation of the present invention preferably has a maximum particle size of less than 300 $\mu$m. (In other words, the microsphere (MS) preparation has no particles with a particle size of 300 $\mu$m or more.) The microsphere (MS) preparation of the present invention more preferably has a maximum particle size of 100 $\mu$m or less, and more

preferably 50 μm or less.

In a preferred embodiment of the invention, the number average particle size of the sustained-release microspheres is within the range of 10 to 50 μm, and preferably within the range of 20 to 40 μm, and the maximum particle size is 100 μm or less.

With the sustained-release microspheres having such a particle size, the lung-specific therapeutic agent of the present invention is selectively trapped in the lungs and can be lung-specific.

The number average particle size and the maximum particle size of sustained-release microspheres used in the present invention can be adjusted by using known methods, such as sifting for selecting sustained-release microspheres having a desired number average particle size and maximum particle size.

For example, the particle size of the sustained-release microspheres can be adjusted by the concentration of the test substance at the time of production, stirring speed, etc.

As long as the object of the present invention is achieved, the amount of the biodegradable polymer may be suitably changed according to the potency of pharmacological activity of the active ingredient, the desired drug release rate, etc. For example, the amount of the biodegradable polymer may be about 0.2 to 10,000 times (mass ratio), preferably about 1 to 1,000 times (mast ratio), more preferably about 1 to 100 times (mass ratio) the amount of the physiologically active substance.

The sustained-release microspheres of the present invention can be produced, for example, by using known methods, such as in-water drying (e.g., o/w, w/o, and w/o/w), phase separation, spray drying, and granulation in supercritical fluid.

For example, methods for producing sustained-release microspheres containing a $PGI_2$ agonist as mentioned above are disclosed in WO2004/032965, Patent No. 4,497,320, and WO2008/047863.

The sustained-release microspheres used in the present invention can be produced by or in accordance with such production methods.

The sustained-release microspheres used in the present invention have a very low toxicity, and are thus highly safe for use as medicine.

The lung-specific therapeutic agent of the present invention comprises the sustained-release microspheres. When the microspheres are lipophilic, it is preferably an o/w emulsion. When the microspheres are hydrophilic, it is preferably w/o emulsion, or w/o/w emulsion.

The lung-specific therapeutic agent of the present invention can be produced, for example, by dispersing the sustained-release microspheres in an aqueous dispersion medium.

Examples of the aqueous dispersion medium include solutions obtained by dissolving in distilled water at least one additive selected from the group consisting of isotonizing agents (e.g., sodium chloride, glucose, mannitol, sorbitol, and glycerol), dispersants (e.g., Tween 80, carboxymethyl cellulose, and sodium alginate), preservatives (e.g., benzyl alcohol, benzalkonium chloride, and phenol), and soothing agents (e.g., glucose, calcium gluconate, and procaine hydrochloride).

The lung-specific therapeutic agent of the present invention is administered by intravenous injection. The therapeutic agent may be administered once or multiple times. The administration period may be, for example, 1 week to 6 months, and preferably 1 to 4 weeks. The frequency of administration may be about once a week to once a month. When the administration is repeated multiple times, intravenous administration is performed after the sustained-release period of the first administration.

The lung-specific therapeutic agent of the present invention is selectively trapped in the lungs and the pharmaceutical compound is gradually released into the lung tissue to exert therapeutic effects.

Accordingly, the intravenous administration may be performed intermittently (for example, at weekly intervals) multiple times while confirming efficacy and safety.

Since even a low-frequency administration of, for example, about once a week to once a month as mentioned above, which places less of a burden on patients, can provide prolonged therapeutic effects, administration compliance can be improved.

As pharmaceutical compounds, in place of Compounds 1, 3, and 5, for example, steroid drugs (beclomethasone: 400 μg/day, inhalation; fluticasone: 200 μg/day, inhalation), a leukotriene receptor antagonist (LTRA) (montelukast: 10 mg/day, oral administration), $M_3$ receptor blockers (tiotropium: 5 μg/day, inhalation, imidafenacin: 200 μg/day, oral administration), a $PGI_2$ derivative (beraprost: 180 μg/day, oral administration), and $\beta_2$-adrenoceptor stimulants (tulobuterol: 2 mg/day, patch; salmeterol: 100 μg/day, inhalation) can be also used to prepare microsphere (MS) preparations with lactic acid-glycolic acid copolymers (PLGA), polylactic acids (PLA), gelatin hydrogels and/or liposomes. In the parenthesis are shown typical therapeutic drugs used in clinical practice as well as typical doses and administration methods.

The clinical dose of the inhalant comprising such a pharmaceutical compound is 5 to 400 μg/day in terms of the active ingredient. The inhalent is administered several times per day. Accordingly, for example, when 400 μg, i.e., the maximum dose in terms of the active ingredient, is administered for 4 weeks, the total dose of the active ingredient

is 11.2 mg. When sustained-release microspheres containing 10% of the active ingredient are used, the total dose in terms of the microspheres is 112 mg. This dose in terms of the sustained-release microspheres is as low as or lower than the no-observed-effect level shown in the Example (Pharmacological Effect Test 1), i.e., 5.8 mg/kg (348 mg/person). This value can be considered to be a safe dose that does not develop small pulmonary embolism when sustained-release microspheres having a particle size of 20 to 40 μm are intravenously administered.

Specifically, the lung-specific therapeutic agent of the present invention can be safely administered since even the single intravenous administration of the pharmaceutical compound at a 4-week-equivalent dose does not develop small pulmonary embolism.

Further, the pulmonary-disease-specific therapeutic agent of the present invention intravenously administered has high lung tissue selectivity that is preferably at least 10 times as high as preparations for systemic administration, such as oral preparations and patches.

In this case, the results of trial calculation of a pharmaceutical compound that is required to be administered at a relatively large dose of 10 mg/day (total 4-week dose is 280 mg) in terms of the active ingredient indicate that, for example, the dose of the lung-specific therapeutic agent of the present invention comprising the sustained-release microspheres of the present invention (assuming a 10-fold lung-selectivity compared to conventional preparations and comprising 10% of the active ingredient) is 280 mg/person in terms of the sustained-release microspheres. This value is also as low as or lower than the no-observed-effect level shown in the Example (Pharmacological Effect 1) mentioned above, i.e., 348 mg/person. Accordingly, in this case also, the lung-specific therapeutic agent of the present invention can be safely administered since even a single intravenous administration of the pharmaceutical compound at a 4-week-equivalent dose, based on the dose of a conventional preparation, does not develop small pulmonary embolism.

The present inventors found that an increased blood $TXA_2$ concentration (measured in terms of the concentration of 11-dehydro-$TXB_2$, which is a metabolite of $TXA_2$) in monocrotaline-induced pulmonary hypertension rat models, compared to normal animals, is the cause of exacerbation. The inventors further found that administration of $PGI_2$ (epoprostenol) further increases the blood $TXA_2$ concentration. Repeated administration of Compound 1 having $PGI_2$ agonistic activity and $TXA_2$ synthetase inhibitory activity inhibits an increase of the blood $TXA_2$ concentration, thus suggesting that repeated administration of Compound 1 does not result in attenuation of (resistance to) effects and confirming its efficacy in survival rate, pulmonary artery pressure, right ventricular hypertrophy, pulmonary artery medial thickening, etc. (Am J Respir Crit Care Med 172, 1575-1580 (2005)).

Repeated administration of a $PGI_2$ agonist (e.g., $PGI_2$ and Compound 3) having platelet aggregation inhibitory activity and vasodilatory activity increases $TXA_2$ production having platelet aggregation inhibitory activity and vasodilatory activity in the living body to maintain a biological balance, thus resulting in attenuation of (resistance to) $PGI_2$ agonistic activity.

The inventors found that repeated administration of a $PGI_2$ agonist in combination with a $TXA_2$ synthetase inhibitor (e.g., ozagrel or a salt thereof) inhibits $TXA_2$ production to inhibit attenuation of (resistance to) $PGI_2$ agonistic activity and maintain its effect, and that a combination drug of a $PGI_2$ agonist and a $TXA_2$ synthetase inhibitor is thus useful as a preparation.

Pharmacological Effect Test 5 shows the effects of Compound 3, Compound 7, and ozagrel hydrochloride administered alone or in combination to pulmonary hypertension models, and compares these individual or combined effects with the effect of Compound 1.

Specifically, the inventors newly found that when an injection of a $PGI_2$ agonist, such as epoprostenol, $PGE_1$ (alprostadil), or a carbacyclin derivative, is administered, administration of an injection of a $TXA_2$ synthetase inhibitor sodium ozagrel (trade name: cataclot) in combination with the $PGI_2$ agonist, or a combination drug thereof is useful. Similarly, the present inventors newly found that when an oral agent of a $PGI_2$ agonist such as beraprost sodium (Compound 3) or a PGE derivative (e.g., ornoprostil (Compound 7), limaprost, enprostil, and misoprostol) is administered, administration of an oral preparation of a $TXA_2$ synthetase inhibitor such as ozagrel hydrochloride (Vega) or a $TXA_2$ receptor antagonist in combination with the $PGI_2$ agonist, or a combination drug thereof is useful.

The dose of the pulmonary-disease-specific therapeutic agent of the present invention varies according to the age, body weight, symptoms, therapeutic effect, administration method, treatment period, etc. In general, the pulmonary-disease-specific therapeutic agent is intravenously administered once a week, once every four weeks, once every three months, or once to several times every six months, at a dose of 1 ng to 1,000 mg per adult in terms of the active substance.

Of course, since the dose depends on various conditions as described above, the dose may be less than the above amount or may need to exceed the above range.

The dose of the lung-specific therapeutic agent of the present invention varies according to the kind and content of pharmaceutical compound as an active ingredient, target disease, etc. The dose is typically 10 mg/kg of the body weight (600 mg/person) or less. From the viewpoint of a high degree of preventing small pulmonary embolism, the lung-specific therapeutic agent is preferably 6 mg/kg of the body weight (or 360 mg/person) or less.

Examples of indications of intravenous therapy using the sustained-release preparation of the present invention include pulmonary diseases such as acute pneumonia, pulmonary fibrosis, interstitial pneumonia, pulmonary hypertension, chronic obstructive pulmonary disease (COPD), chronic bronchitis, pulmonary emphysema, asthma, intractable asthma, systemic inflammatory response syndrome (SIRS), acute lung injury (ALI), acute respiratory distress syndrome (ARDS), sarcoidosis, chronic idiopathic pulmonary thromboembolism, diffuse panbronchiolitis, hypersensitivity pneumonia, lung cancer, obesity hypoventilation syndrome, alveolar hypoventilation syndrome, and chronic rejection in lung transplantation.

The lung-specific therapeutic agent of the present invention may be used in combination with other medicaments or therapeutic methods in order to complement or enhance therapeutic effects, improve kinetics and absorption, reduce the dose, alleviate side effects, etc. The other drugs or therapeutic methods include not only those that have so far been found but also those that will be found in the future.

The lung-specific therapeutic agent of the present invention and one or more other components or drugs may be administered as a combination preparation comprising these components, or may be administered separately. When the lung-specific therapeutic agent of the present invention and one or more other drugs are administered separately as independent preparations, they may be administered simultaneously or with time lag. Administration with time lag includes the method of administering the sustained-release preparation of the present invention before the one or more other preparations, and vice versa, and each administration methodmay be the same or different.

The other drugs may be, for example, low molecular weight compounds or high molecular weight compounds, such as proteins, polypeptides, polynucleotides (DNA, RNA and genes), antisenses, decoys, antibodies, vaccines, stem cells isolated from tissue, iPS cells, or somatic cells, etc. As the other drugs, an oral preparation of Compound 1, 3, or 5 may be repeatedly administered, or a sustained-release preparation of Compound 1, 3, or 5 may be intermittently administered subcutaneously or intramuscularly. The dose of the other drugs can be appropriately selected based on the clinically used dose as a reference. The blending ratio of the therapeutic agent of the present invention with one or more other drugs can be appropriately selected according to the age and body weight of the subject to whom the therapeutic agent is administered, administration method, administration time, disease to be treated, symptoms, combination, and the like. For example, the one or more other drugs may be used in an amount of 0.01 to 100 parts by mass per part by mass of the therapeutic agent concurrently used in the present invention. One or more types of drugs selected from the same group or different groups described below may be administered alone or in combination at a suitable ratio.

When the sustained-release microsphere preparation is intravenously administered repeatedly, or two or more types of preparations are administered multiple times, the subsequent administration is performed after the sustained-release period of the preparation administered first has finished.

There is no limitation on the diseases on which the combination preparations of the pulmonary-disease-specific therapeutic agent of the present invention and one or more other drugs have preventive and/or treatment effects, as long as the preventive and/or therapeutic effect of the pulmonary-disease-specific therapeutic agent of the present invention on the disease is complemented and/or enhanced by the other drugs.

According to another preferred embodiment of the present invention, the lung-specific therapeutic agent comprises sustained-release microspheres containing at least one pulmonary disease therapeutic drug selected from the group consisting of (E)-[5-[2-[1-phenyl-1-(3-pyridyl)methylideneaminooxy]ethyl]-7,8-dihydronaphthalen-1-yloxy]acetic acid (Compound 1), (±)-(1R,2R,3aS,8bS)-2,3,3a,8b-tetrahydro-2-hydroxy-1-[(E)-(3S,4RS)-3-hydroxy-4-methyl-1-octen-6-inyl]-1H-cyclopenta[b]benzofuran-5-butanoic acid (beraprost), 2-{4-[N-(5,6-diphenylpyrazin-2-yl)-N-isopropylamino]butyloxy}acetic acid (MRE-269) (Compound 5), and

salts thereof; and

a biodegradable polymer selected from the group consisting of polylactic acids, lactic acid-glycolic acid copolymers, and mixtures thereof.

According to another preferred embodiment of the present invention, the lung specific-disease therapeutic agent comprises sustained-release microspheres containing at least one pulmonary disease therapeutic drug selected from the group consisting of beclomethasone, fluticasone, montelukast, tiotropium, imidafenacin, beraprost, carbacyclin, sivelestat, tulobuterol, salmeterol; (E)-[5-[2-[1-phenyl-1-(3-pyridyl)methylideneaminooxy]ethyl]-7,8-dihydronaphthalen-1-yloxy]acetic acid (Compound 1); (±)-(1R,2R,3aS,8bS)-2,3,3a,8b-tetrahydro-2-hydroxy-1-[(E)-(3S,4RS)-3-hydroxy-4-methyl-1-octen-6-inyl]-1H-cyclopenta[b]benzofuran-5-butanoic acid (Beraprost); 2-{4-[N-(5,6-diphenylpyrazin-2-yl)-N-isopropylamino]butyloxy}acetic acid (Compound 5); carbacyclin derivatives that are $PGI_2$ derivatives; and salts thereof, the sustained-release microspheres having a number average particle size of 20 to 40 μm and a maximum particle size of 100 μm or less, and

is administered at a dose of 6 mg/kg or less in terms of the sustained-release microspheres.

Furthermore, the therapeutic agent of the invention, which is lung-specific, can be expected to, for example, reduce the total dose, enhance therapeutic effects, alleviate side effects, improve administration compliance, and achieve economic effects.

Examples

Methods for producing preparations and pharmacological tests are described below as examples of the present invention in order to provide a better understanding of the present invention, and should not be understood to limit the present invention.

1. Production of Sustained-release MS

Preparation Example 1: Sustained-release MS of Compound 1

A dichloromethane/methanol (9.4 mL) solution containing 670 mg of a lactic acid-glycolic acid copolymer ("PLGA") (polylactic acid:glycolic acid = 1:1 (mol %), weight average molecular weight 50,000, PLGA5-50, Mitsui Chemicals, Inc.) and Compound 1 (170 mg; Sigma Co., No. 02264) was prepared. An O/W emulsion was prepared by adding the solution prepared above to 2 L of a 0.1% polyvinyl alcohol (Nacalai Tesque, Inc.) aqueous solution (pH 3.0, adjusted with 1N hydrochloric acid) which had been stirred at 3,500 rpm using a homogenizer (T.K. homomixer, Primix Corporation), and stirring the resulting mixture at room temperature for 1 minute. This O/W emulsion was stirred at room temperature for 4 hours to evaporate dichloromethane, and the oil phase was solidified and then centrifuged at 3,000 rpm for 10 minutes using a centrifuge (O5PR-22, Hitachi Ltd.). After the supernatant was removed and the residue was dispersed in distilled water for injection (35 ml), the dispersion was centrifuged at 3,000 rpm for 10 minutes using the centrifuge. After the supernatant was removed and the residue was dispersed in 0.2% Tween 80 solution (35 ml), the dispersion was centrifuged at 3,000 rpm for 10 minutes using the centrifuge. After the supernatant was removed and the residue was dispersed in distilled water for injection (35 ml), the dispersion was centrifuged again at 3,000 rpm for 10 minutes using the centrifuge. After the supernatant was removed and the precipitate was dispersed again with a small amount of distilled water, the dispersion was freeze-dried using a lyophilizer (FZ-6PV, Labconco Corporation) to produce a microsphere (MS) preparation of Compound 1.

As the MS of Preparation Example 1, microspheres (MS) (Compound 1-MS) having a Compound 1 content of 14.9%, a number average particle size of 30.3 μm, and a maximum particle size of 100 μm or less were thus obtained. The content of Compound 1 and particle size of MSwere determined by using the methods described below. The same applies to the following Preparation Examples.

Preparation Example 2: Negative Control

A dichloromethane/methanol (9.4 mL) solution of 670 mg of a lactic acid-glycolic acid copolymer ("PLGA") (polylactic acid:glycolic acid = 1: 1 (mol %), weight average molecular weight 50,000, PLGA5-50, Mitsui Chemicals, Inc.) was prepared. The same procedure as in Preparation Example 1 was followed to produce microspheres (MS) of PLGA.

As the MS of Preparation Example 2 (a negative control), microspheres (MS) (PLGA-MS (negative control)) containing no pharmaceutical compound and having a number average particle size of 33.8 μm and a maximum particle size of 100 μm or less were thus obtained.

Preparation Example 3:Sustained-release MS of Compound 3

A dichloromethane/methanol (4 mL) solution containing 40 mg of a lactic acid-a glycolic acid copolymer (hereinafter referred to as "PLGA")(polylactic acid:glycolic acid = 1:1 (mol %), weight average molecular weight 50,000, PLGA5-50, Mitsui Chemicals, Inc.) and Compound 3 (10 mg; Cayman Chemical Co., No.18230) was prepared. An O/W emulsion was prepared by adding the solution prepared above to 100 mL of a 0.1% polyvinyl alcohol (Nacalai Tesque, Inc.) aqueous solution (pH 3.0, adjusted with 1N hydrochloric acid) which had been stirred at 3,000 rpm using a TK Robomix (MARK II 2.5 Model, Tokushu Kiki Co., Ltd.), and stirring the resulting mixture at room temperature for 0.5 minutes. This O/W emulsion was stirred at room temperature for 4 hours to evaporate dichloromethane, and the oil phase was solidified and then centrifuged at 3,000 rpm for 10 minutes using a centrifuge (O5PR-22, Hitachi Ltd.). After the supernatant was removed and the residue was dispersed in distilled water for injection (35 mL), the dispersion was centrifuged at 3,000 rpm for 10 minutes using the centrifuge. After the supernatant was removed and the residue was dispersed in 0.2% Tween 80 solution (35 mL), the dispersion was centrifuged at 3,000 rpm for 10 minutes using the centrifuge. After the supernatant was removed and the residue was dispersed in distilled water for injection (35 mL), the dispersion was centrifuged again at 3,000 rpm for 10 minutes using the centrifuge. After the supernatant was removed, the precipitate was soaked in dry ice-methanol, frozen, and then dried under reduced pressure to produce microspheres (MS) of Compound 3.

As the MS of Preparation Example 3, microspheres (MS) (Compound 3-MS) having a Compound 3 content of 8.9%, a number average particle size of 24.9 μm, and a maximum particle size of 100 μm or less were thus obtained.

Preparation Example 4: Sustained-release MS of Compound 5

A dichloromethane/methanol (4 mL) solution containing 40 mg of a lactic acid-a glycolic acid copolymer (hereinafter referred to as "PLGA") (polylactic acid:glycolic acid = 1:1 (mol %), weight average molecular weight 50,000, PLGA5-50, Mitsui Chemicals, Inc.) and Compound 5 (10 mg; Cayman Chemical Co., No. 10010412) was prepared. An O/W emulsion was prepared by adding the solution prepared above to 100 mL of a 0.1% polyvinyl alcohol (Nacalai Tesque, Inc.) aqueous solution (pH 3.0, adjusted with 1N hydrochloric acid) which had been stirred at 3,000 rpm using a TK Robomix (MARK II 2.5 Model, Tokushu Kiki Co., Ltd.), and stirring the resulting mixture at room temperature for 0.5 minutes. This O/W emulsion was stirred at room temperature for 2 hours to evaporate dichloromethane, and the oil phase was solidified and then centrifuged at 3,000 rpm for 10 minutes using a centrifuge (O5PR-22, Hitachi Ltd.). After the supernatant was removed and the residue was dispersed in distilled water for injection (2 mL), the dispersion was centrifuged at 3,000 rpm for 10 minutes using the centrifuge. After the supernatant was removed and the residue was dispersed in 0.2% Tween 80 solution (35 mL), the dispersion was centrifuged at 3,000 rpm for 10 minutes using the centrifuge. After the supernatant was removed and the residue was dispersed in distilled water for injection (2 ml), the dispersion was centrifuged again at 3,000 rpm for 10 minutes using the centrifuge. Finally, after the supernatant was removed, the precipitate was soaked in dry ice-methanol, frozen, and then dried under reduced pressure to produce microspheres (MS) of Compound 5.

As the MS of Preparation Example 4, microspheres (MS) (Compound 5-MS) having a Compound 5 content of 16.3%, a number average particle size of 35.3 μm, and a maximum particle size of 100 μm or less were thus obtained.

Preparation Test Example 1: Measurement of Encapsulation Efficiency

An acetonitrile solution containing an appropriate internal standard was added to the microspheres produced in Preparation Examples 1, 3, and 4 (about 10 mg each), and each of the resulting mixtures was subjected to sonication to dissolve the microspheres. The Compound 1 content of each of the solutions was measured by a high performance liquid chromatography (HPLC), and encapsulation efficiency of Compounds 1, 3 and 5 in microspheres was calculated by the following formula.

$$\text{Encapsulation efficiency (\%)} = (\text{Content measured/Theoretical Content}) \times 100$$

As a result, all of the microspheres of Preparation Examples 1, 3, and 4 had an encapsulation efficiency of 70% or more, a compounds content of 8% to 18%, and a number average particle size of 24 to 36 μm. These preparations contained no microspheres having a number average particle size of 100 μm or more.

Preparation Test Example 2: In vitro Release Test and Particle Size Measurement

3 mg of each of the microspheres (MS) produced in Preparation Examples 1, 3, and 4 was weighed for each sampling point (n=3). After 10 mL of a 0.2% (w/v) Tween 80-containing 1/15 M phosphate buffer (pH 7) was added thereto, the microspheres were uniformly dispersed by vortexing (10 seconds) and sonication (20 seconds), and the dispersion was allowed to stand in a 37°C water bath. Each container as a whole was sampled over time and centrifuged (at 2,000 rpm for 5 minutes), and 4 mL of the supernatant and the pellet obtained by discarding the remaining supernatant were stored frozen.

After 10 mL of DMSO was added to this pellet, the microspheres (MS) were fully dissolved by vortexing (10 seconds). To 300 μL of this solution were added 200 μL of IS solution B and 500 μL of solution of mobile phase (pH 3). The resulting mixture was mixed well. Similarly, to 300 μL of the supernatant were added 200 μL of IS solution B and 500 μL of solution of mobile phase (pH of 3). The resulting mixture was mixed well. After the mixture was centrifuged (12,000 rpm, 3 minutes), 10 μL of the supernatant was injected on HPLC.

HPLC Conditions

Devices: Chromatograph (Shimazu LC-10AT), UV detector (Shimazu SPD-10A), data analyzer (Shimazu C-R7A)
Detection: UV 265-nm
Column: SHISEIDO CAPCELLPACK C18 UG120 (4.6 mm i.d. x 150 mm)
Column temperature: a constant temperature around 25°C
Mobile phase: acetonitrile:water:triethylamine = 1000:900:3 (an aqueous triethylamine solution at a water:triethylamine ratio of 900:3 was adjusted to a pH of 3 with phosphoric acid)

Flow rate: 1.0 mL/min
Internal standard (IS): n-Propylparaben

• Particle Size Measurement

The particle size was measured with a Coulter counter (Multisizer III, Beckman Coulter, Inc., USA).
Fig. 1 shows the results of release of Compound 1 from the microspheres (MS) produced in Preparation Example 1. Fig. 2 shows the results of release of Compound 3 from the microspheres (MS) produced in Preparation Example 3. Fig. 3 shows the results of release of Compound 5 from the microspheres (MS) produced in Preparation Example 4.
The results show that the MS (Compound 1-MS) of Preparation Example 1 released 90% or more in about 4 weeks, the MS (Compound 3-MS) of Preparation Example 3 released 90% or more in about 2 weeks, and the MS (Compound 5-MS) of Preparation Example 4 released 85% or more in about 2 weeks.

Preparation Test Example 2: In Vivo Release Test

Blood kinetics were determined using SD male rats (SPF) provided from Japan SLC, Inc. (Hamamatsu). Using a 23G disposable injection needle (Terumo Corporation) and a disposable injection syringe with a capacity of 2.5 mL (Terumo Corporation), the suspension was subcutaneously administered once into the dorsal region of each rat at a dose of 10 mg/kg in terms of Compound 1. The amount of administration was 5 mL/kg. The number of mice in each group was five.
Using a disposable heparinized syringe with a 23G injection needle, 0.5 mL of blood was collected from the jugular vein at each blood sampling point. After the blood was centrifuged (12,000 rpm, 10 minutes, 4°C), the plasma was kept frozen (-30°C). After the test, the blood concentration of Compound 1 was determined by LC/MS/MS.

LC/MS/MS Assay

MS/MS Conditions
MS/MS: API 4000
Ionization mode: ESI
Ion polarity mode: positive
Monitor ion: (see Table 1)

Table 1

| Compound | Precursor ion* (m/z) | Product ion* (m/z) |
|---|---|---|
| Compound 1 | 429.2 | 79.1 |
| Internal standard (IS) | 445.4 | 168.1 |
| *: The highest ionic strength value within m/z ±0.5 m/z relative to the target value was suitably selected. | | |

Results: Fig. 4 shows blood kinetics after subcutaneous administration of Preparation Example 1 to the rats. The blood kinetics of Preparation Example 1 were prolonged for about 4 weeks.

Pharmacological Effect Test 1: Test on Safety in Intravenous Administration of Preparation Example 2 (PLGA-MS (Negative Control))

After Slc;Wistar male rats (5 weeks old) were subjected to inhalation anesthesia with isoflurane, an aqueous monocrotaline (MCT) solution was subcutaneously administered to the dorsal region of each rat at a dose of 60 mg/kg (an injection volume of 3 mL/kg) using a disposable injection syringe and a 27G disposable injection needle to prepare pulmonary hypertension models. The day of the model preparation was defined as day 0. Immediately after the MCT administration, PLGA-MS of Preparation Example 2 (negative control) was subcutaneously administered at various doses to evaluate 42-day survival rate.
The test substances of groups 2 to 6 were suspended in the same medium as used in Group 1 (0.2 w/v% Tween 80 physiological saline) and intravenously administered at 10 mL/kg.
In the below tests, the test substances were suspended in the medium and intravenously or subcutaneously administered in the same way.
Table 2 shows the test group constitution.

Table 2

| Group | Administered substance | Dose* | Number of animals |
|---|---|---|---|
| 1 | 0.2 w/v% Tween 80 physiological saline | 10 mL/kg | 10 |
| 2 | PLGA-MS (negative control) | 0.3 mg equivalent/kg (1.74 mg/kg) | 10 |
| 3 | PLGA-MS (negative control) | 1 mg equivalent/kg (5.8 mg/kg) | 10 |
| 4 | PLGA-MS (negative control) | 3 mg equivalent/kg (17.4 mg/kg) | 10 |
| 5 | PLGA-MS (negative control) | 10 mg equivalent/kg (58 mg/kg) | 10 |
| 6 | PLGA-MS (negative control) | 0.1 mg equivalent/kg (0.58 mg/kg) | 10 |
| *: The doses indicate the amounts (equivalents) of Compound 1 assuming that Preparation Example 2 contains Compound 1 in the same amount (17.4%) as Preparation Example 1. The amounts in parentheses indicate the doses of PLGA-MS actually administered. | | | |

Group 1: Immediately after the model preparation and 21 days after the model preparation, 0.2 w/v% Tween 80 physiological saline (medium) was intravenously administered intermittently at a dose of 10 mL/kg (administered twice in total). The survival rate over a 42-day period after the model preparation was evaluated.

Group 2: Immediately after the model preparation and 21 days after the model preparation, PLGA-MS was intravenously administered intermittently at a dose of 1.74 mg/kg (administered twice in total) . The survival rate over a 42-day period after the model preparation was evaluated.

Group 3: Immediately after the model preparation and 21 days after the model preparation, PLGA-MS was intravenously administered intermittently at a dose of 5.8 mg/kg (administered twice in total) . The survival rate over a 42-day period after the model preparation was evaluated.

Group 4: Immediately after the model preparation and 21 days after the model preparation, PLGA-MS was intravenously administered intermittently at a dose of 17.4 mg/kg (administered twice in total) . The survival rate over a 42-day period after the model preparation was evaluated.

Group 5: Immediately after the model preparation and 21 days after the model preparation, PLGA-MS was intravenously administered intermittently at a dose of 58 mg/kg (administered twice in total) . The survival rate over a 42-day period after the model preparation was evaluated.

Group 6: Immediately after the model preparation and 21 days after the model preparation, PLGA-MS was intravenously administered intermittently at a dose of 0.58 mg/kg (administered twice in total). The survival rate over a 42-day period after the model preparation was evaluated.

Fig. 5 shows the survival rate curves.

The survival rate of the medium-administered group (Group 1) was 20%.

The survival rates of some negative control groups receiving PLGA-MS, i.e., 0.58 mg/kg group (Group 6), 1.74 mg/kg group (Group 2), and 5.8 mg/kg group (Group 3) were equivalent to that of the medium-administered group (Group 1).

In contrast, the 17.4 mg/kg group (Group 4) and 58 mg/kg group (Group 5) receiving PLGA-MS exhibited a tendency to be shorter survival rate than the medium-administered group (Group 1), 0.58 mg/kg group (Group 6), 1.74 mg/kg group (Group 2), and 5.8 mg/kg group (Group 3).

The above results confirmed that the nontoxic dose of intravenously administered PLGA/MS is 5.8 mg/kg (1 mg/kg in terms of Compound 1) or less. The dose of 5.8 mg/kg corresponds to 348 mg/kg of a person, which is a dose difficult for intravenous administration.

This pulmonary hypertension model dies due to right heart failure which is developed by increasing resistance to pulmonary blood flow and elevating pulmonary arterial pressure associated with obstruction of micro pulmonary arteries in lung tissue. Thus, this pulmonary hypertension model is highly sensitive to pulmonary embolism. Pulmonary fibrosis, asthma, COPD, and the like are diseases of bronchi and pulumonary alveolar tissue. Patients with these diseases are less sensitive to pulmonary (micro)embolism and safer, compared to patients with pulmonary hypertension and collagen diseases that have resistance to pulmonary blood flow.

Pharmacological Effect Test 2: Investigation of the Relationship between Survival Benefit and Dose in Intravenous Administration of Preparation Example 1 (Compound 1-MS)

An evaluation was performed using the same model system as in Pharmacological Effect Test 1. Table 3 shows the test group constitution.

Table 3

| Group | Administered substance | Dose* | Number of animals |
|---|---|---|---|
| 1 | PLGA-MS (negative control) | 1 mg equivalent/kg | 10 |
| 2 | Compound 1-MS | 0.03 mg/kg | 10 |
| 3 | Compound 1-MS | 0.1 mg/kg | 10 |
| 4 | Compound 1-MS | 0.3 mg/kg | 10 |
| 5 | Compound 1-MS | 1 mg/kg | 10 |
| *: The dose of PLGA-MS (negative control) (Preparation Example 2) in Group 1 was determined from the results of Pharmacological Effect Test 1. PLGA-MS was administered to Group 1 in an amount equivalent to the amount administered to Group 5. The doses to Groups 2 to 5 are indicated in terms of Compound 1. | | | |

Group 1: Immediately after the model preparation and 21 days after the model preparation, Preparation Example 2 (PLGA-MS) was intravenously administered intermittently at a dose of 5.8 mg/kg (1 mg/kg equivalent of Compound 1) (administered twice in total). The survival rate over a 42-day period after the model preparation was evaluated.
Group 2: Immediately after the model preparation and 21 days after the model preparation, Preparation Example 1 (Compound 1-MS) was intravenously administered intermittently at a dose of 0.03 mg/kg (in terms of Compound 1) (administered twice in total). The survival rate over a 42-day period after the model preparation was evaluated.
Group 3: Immediately after the model preparation and 21 days after the model preparation, Preparation Example 1 (Compound 1-MS) was intravenously administered intermittently at a dose of 0.1 mg/kg (in terms of Compound 1) (administered twice in total). The survival rate over a 42-day period after the model preparation was evaluated.
Group 4: Immediately after the model preparation and 21 days after the model preparation, Preparation Example 1 (Compound 1-MS) was intravenously administered intermittently at a dose of 0.3 mg/kg (in terms of Compound 1) (administered twice in total). The survival rate over a 42-day period after the model preparation was evaluated.
Group 5: Immediately after the model preparation and 21 days after the model preparation, Preparation Example 1 (Compound 1-MS) was intravenously administered intermittently at a dose of 1 mg/kg (in terms of Compound 1) (administered twice in total). The survival rate over a 42-day period after the model preparation was evaluated.

Fig. 6 shows results of the survival rate curve.

The survival rate of the negative control group (Group 1) receiving 5.8 mg/kg (1 mg/kg equivalent of Compound 1) of PLGA-MS was 20%.

The survival rates of the 0.03 mg/kg group (Group 2) and 0.1 mg/kg (Group 3) each receiving Preparation Example 1 (Compound 1-MS) were 40%. These groups exhibited a tendency to be prolonged survival rate, compared to the group (Group 1) receiving 5.8 mg/kg (1 mg/kg equivalent of Compound 1) of PLGA-MS.

Further, the 0.3 mg/kg group (Group 4) and 1 mg/kg group (Group 5) receiving Preparation Example 1 (compound 1-MS) exhibited prolonged survival rate, compared to the group (Group 1) receiving 5.8 mg/kg (1 mg/kg equivalent of Compound 1) of Preparation Example 2 (PLGA-MS).

Thus, the results show that when Compound 1 of Preparation Example 1 was intravenously administered intermittently at a dose of 0.03 mg/kg to 1 mg/kg, a dose-dependent survival prolongation effect was provided.

Pharmacological Effect Test 3: Analytical Comparison Test betweenIntravenous and Subcutaneous Administration of Preparation Example 1 (Compound 1-MS) using Animals Slaughtered during the Experiment Priod

An evaluation was performed using the same model system as in Pharmacological Effect Test 1. Table 4 shows the test group constitution.

Table 4

| Group | Administered substance | Dose* | Number of animals |
|---|---|---|---|
| 1 | 0.2 w/v% Tween 80 physiological saline (normal); intravenous administration | 10 mL/kg | 10 |
| 2 | PLGA-MS (negative control); intravenous administration | 1 mg equivalent/kg | 15 |
| 3 | Preparation Example 1 (Compound 1-MS); intravenous administration | 1 mg/kg | 15 |
| 4 | Preparation Example 1 (Compound 1-MS); subcutaneous administration | 1 mg/kg | 15 |
| 5 | Preparation Example 1 (Compound 1-MS); subcutaneous administration | 10 mg/kg | 15 |
| *: The dose of Preparation Example 2 (PLGA-MS) to Group 2 was determined from the results of Pharmacological Effect Tests 1 and 2. | | | |

The doses in Groups 3 to 5 are indicated in terms of Compound 1 contained in the preparations.

Group 1: Normal control group: Water for injection (Japanese Pharmacopoeia) was administered in place of monocrotaline administration. Immediately after preparation of the normal model, 0.2 w/v% Tween 80/physiological saline was intravenously administered a single time. The evaluation was made 21 to 25 days after the model preparation.
Group 2: Negative control: Immediately after the model preparation, Preparation Example 2 (PLGA-MS) was intravenously administered at a dose of 5.8 mg/kg (1 mg/kg equivalent of Compound 1) a single time. The evaluation was made 21 to 25 days after the model preparation.
Group 3: Immediately after the model preparation, Preparation Example 1 (Compound 1-MS) was intravenously administered at a dose of 1 mg/kg (in terms of Compound 1) a single time. The evaluation was made 21 to 25 days after the model preparation.
Group 4: Immediately after the model preparation, Preparation Example 1 (Compound 1-MS) was subcutaneously administered at a dose of 1 mg/kg (in terms of Compound 1) a single time. The evaluation was made 21 to 25 days after the model preparation.
Group 5: Positive control group: Immediately after the model preparation, Preparation Example 1 (Compound 1-MS) was subcutaneously administered at a dose of 10 mg/kg (in terms of Compound 1) a single time. The evaluation was made 21 to 25 days after the model preparation.

The pulmonary artery pressure (right ventricular pressure), the ratio of right ventricular pressure to left ventricular pressure, the ratio of right ventricular weight to left ventricular plus septal weight (RV/ left ventricular plus septal weight) of all the animals alive were determined 21 to 25 days after the administration of MCT.
Further, the Compound 1 contents of the lungs in Groups 3 and 4 were measured.

Measurement Methods: Methods for Determining the Pulmonary Artery Pressure (Right Ventricular Pressure) and Left Ventricular Pressure

After each test animal was anesthetized with pentobarbital sodium (64.8 mg/kg, i.p.), the animal was endotreacheally intubated and connected to a ventilator. The chest was opened by median sternotomy, and a catheter (Miller, Micro-Tip Catheter, Transducer Size; 1.4 Fr) was inserted into the intraventriclars from apices of the right and left ventricles to directly measure the pulmonary artery pressure (right ventricular pressure) and left ventricle pressure. The blood pressure was measured by connecting the catheter and a blood pressure transducer and using an amplifier for blood pressure measurement.
Figs. 7, 8, and 9 show the results.
Compared to the normal group (Group 1), the group with Preparation Example 2 intravenously administered (PLGA-MS) to MCT-administered animals (negative control; Group 2) was significantly high in pulmonary artery pressure (right ventricular pressure), the ratio of right ventricular pressure to left ventricular pressure, and the ratio of right ventricular weight to left ventricular plus septal weight, and exhibited symptoms of pulmonary hypertension, whereas the group to which Preparation Example 1 (Compound 1-MS) was intravenously administered at 1 mg/kg (in terms of Compound 1)

(Group 3) a single time was significantly low in pulmonary artery pressure (right ventricular pressure), the ratio of right ventricular pressure to left ventricular pressure, and the ratio of right ventricular weight to left ventricular plus septal weight. In contrast, the group to which Preparation Example 1 (compound-MS) was subcutaneously administered at 1 mg/kg (in terms of Compound 1) a single time (Group 4) exhibited no effects and was almost equivalent to Group 2 (negative control group) in the pulmonary artery pressure (right ventricular pressure), the ratio of right ventricular pressure to left ventricular pressure, and the ratio of right ventricular weight to left ventricular plus septal weight, whereas the group to which Preparation Example 1 (Compound 1-MS) was subcutaneously administered at 10 mg/kg (in terms of Compound 1) a single time (Group 5) exhibited effects almost equivalent to those achieved by the group to which Preparation Example 1 (Compound 1/MS) was intravenous administered at 1 mg/kg (in terms of Compound 1) a single time (Group 3).

The pulmonary-disease-specific therapeutic effect achieved by intravenous administration of the microsphere (MS) preparation was at least 10 times as potent as that of the subcutaneous administration.

Method for Determining Compound 1 Content of the Lungs in Pharmacological Effect Test 3

A four-fold amount of a mixture of distilled water and ethanol relative to the lung weight was added to form a homogenate. Acetonitrile was added to a part of the homogenate and the resulting mixture was stirred. The centrifuged supernatant was collected and concentrated. After the concentrate was dissolved in a solvent, LC/MS/MS measurement was performed under the same conditions as in Preparation Test Example 2 to determine Compound 1 content of the lungs.

The results (Table 5) show that no concentration of Compound 1 was detected from any sample in Group 2 (control), whereas the average concentration in lung tissue was 76.35 ng/g in Group 3 and the average concentration in lung tissue was 0.659 ng/g in Group 4, and that the ratio of Group 3 to Group 4 was 115.9. More specifically, compared to the subcutaneous injection, the intravenous administration achieved an about 120-fold concentration in lung tissue, and lung selectivity was thus confirmed.

Table 5

| Group | Concentration of Compound 1 in the tissue (ng/g) |
|---|---|
| Group 2 (control) | 0 |
| | 0 |
| | 0 |
| | 0 |
| Group 3 (intravenous administration; 1 mg/kg) | 111 |
| | 50.0 |
| | 99.2 |
| | 45.2 |
| Group 4 (subcutaneous administration; 1 mg/kg) | 1.06 |
| | 0.258 |

Pharmacological Effect Test 4: Efficacy of Subcutaneous Administration of Preparation Example 1 on COPD

Short-term COPD models were prepared by endotracheally administering a cigarette smoke solution and LPS (lipopolysaccharide) to guinea pigs, and the effects of Preparation Example 1 on respiratory functions, pulmonary functions, and changes in lung tissue were investigated using the models.

In accordance with the Biol Pharm. Bull. 2009, 32(9): 1559-1564, a cigarette smoke solution was endotracheally administered to guinea pigs for 16 days, and LPS was endotracheally administered for 3 days (Days 1 to 19) to prepare COPD models. On Day 1, Preparation Example 1 was subcutaneously administered at a dose of 10 mg/kg in terms of Compound 1 a single time.

Specifically, the cigarette smoke solution was prepared by bubbling the mainstream smoke of one cigarette (trade name Hi-lite, Japan Tobacco Inc.) per mL of physiological saline. LPS (lipopolysaccharide; Wako Pure Chemical Industries) was dissolved by adding physiological saline to prepare a solution at a concentration of 500 μg/mL. The thus obtained cigarette smoke solution and LPS were each cryopreserved. As the guinea pigs, Std:Hartley (male) guinea

pigs (seven weeks old) (Japan SLC, Inc.), were used.

On Day 1 to Day 4, Day 6 to Day 9, Day 11 to Day 14, and Day 16 to Day 19, the cigarette smoke solution was intratracheally administered to the guinea pigs at a dose of 200 μL/animal by using a device for intratracheal administration. On Day 5, Day 10, and Day 15, the LPS solution (500 μg/mL) was intratracheally administered to the guinea pigs at a dose of 200 μL/animal.

On Days 1 to 19, physiological saline was intratracheally administered to a normal group at a dose of 200 μL/animal once a day. The normal group, control group, and group to which Preparation Example 1 was administered received a single subcutaneous administration on Day 1 and were evaluated on Day 20. The medium alone was subcutaneously administered to the normal group and the control group a single time, whereas Preparation Example 1 was subcutaneously administered at a dose of 10 mg/kg (in terms of Compound 1) a single time to the group receiving Preparation Example 1.

Respiratory functions (airway resistance, tidal volume) in guinea pigs were measured while awake by double-flow plethysmography using a general respiratory function analysis system (Pulmos-I, MIPS, Inc.). One hundred tidal breaths of each guinea pig were measured and the average was defined as the measurement value of each measurement date.

Airway resistance is mainly an indicator of bronchoconstriction and airway obstruction. The tidal volume indicates the amount of air inhaled at resting ventilation and is an indicator of gas exchange and of airway obstruction.

The measurement of pulmonary functions was performed by inserting and fixing a tube in intaratrachea of guinea pigs under urethane anesthesia (1.2 g/kg body weight; i.p.) and measuring the pulmonary functions (residual volume, functional residual capacity) of the guinea pigs.

The "functional residual capacity" refers to the volume of air remaining in the lungs under eupneaand is an indicator of overdistention. The "residual volume" refers to the volume of air present in the lungs at maximum exhalation and is an indicator of having COPD, just like functional residual capacity.

Measurement of Mean Linear Intercepts of Pulmonary Alveoli and Pathology Evaluation: Neutral buffered 10% formalin was infused through a tube inserted in intratrachea at a pressure of 25 cm $H_2O$ for 4 hours. The tissue was then immersed in 10% neutral buffered formalin and fixed for 24 hours, followed by HE staining in a usual manner, thus preparing a tissue sample. The tissue sample was subjected to a histological examination under a microscope.

Images of pulmonary alveoli of the HE-stained tissue sample were photographed with a digital camera. The photographed images were captured in an Olympus image analyzer (DP 70, Olympus Corporation) to measure the mean linear intercepts (MIL) of pulmonary alveoli. A grid of fifty lines of equal spacing and equal length was overlaid onto each image. The grid length to be analyzed was defined as a total extension of 10 mm (constant).

In the portion within the field of view from which the pleural portion, blood vessels, and peripheral airway were excluded, the number of pulmonary alveolar walls crossing one grid square was counted. The grid length was divided by the number of pulmonary alveolar walls crossing one grid square to calculate MLI. The MLI of ten fields of view was determined per sample, and the average was defined as the MLI of the sample.

Table 1 shows the measurement results on Day 20.

Table 6

| | Normal group | Control group | Preparation Example 1 (single subcutaneous administration) |
|---|---|---|---|
| Airway resistance ($cmH_2O \cdot sec$) | 1.367 ± 0.084 | 2.114 ± 0.141 | 1.501 ± 0.110 |
| Tidal volume (mL) | 3.165 ± 0.186 | 2.897 ± 0.107 | 3.544 ± 0.230 |
| Residual volume (mL) | 3.527 ± 0.280 | 5.683 ± 0.510 | 3.969 ± 0.418 |
| Functional residual capacity (mL) | 7.274 ± 0.189 | 8.768 ± 0.246 | 7.572 ± 0.405 |
| Mean linear intercepts of pulmonary alveoli (μm) | 45.5 ± 0.9 | 51.9 ± 0.8 | 47.0 ± 1.1 |

The normal group had an airway resistance (sRaw) of around 1.367 $cmH_2O \cdot sec$ on Day 20.

The control group had a sRaw of 2.114 $cmH_2O \cdot sec$ on Day 20, which is a significantly high value on Day 20 compared to the normal group.

In contrast, the group to which Preparation 1 was administered had a sRaw of 1.501 $cmH_2O$·sec on Day 20, which is a significantly low value compared to the control group.

The normal group had a tidal volume (TV) of around 3.165 mL. The TV of the control group was reduced to 2.897 mL, but no significant difference compared to the normal group was observed. On the other hand, the group of single subcutaneous administration of Preparation Example 1 had a TV of 3.544 mL, which is a significantly high value compared to the control group.

The normal group had a residual volume (RV) of 3.527 mL, whereas the control group had a RV of 5.683 mL, which is a significantly high value compared to the normal group. In contrast, the group of single subcutaneous administration of Preparation Example 1 was 3.969 mL, which is a significantly low value compared to the control group.

The normal group had a functional residual capacity (FRC) of 7.274 mL. The control group had an FRC of 8.768 mL, which is a significantly high value compared to the normal group. In contrast, the group of single subcutaneous administration of Preparation Example 1 had an FRC of 7.572 mL, which is a significantly low value compared to the control group.

The normal group had mean linear intercepts (MLI) of pulmonary alveoli of 45.5 μm.

The control group had an MLI of 51.9 μm, which is a significantly high value compared to the normal group. In contrast, the group of single subcutaneous administration of Preparation Example 1 had an MLI of 47.0 (μm, which is a significantly low value compared to the control group, thus confirming inhibition of pulmonary alveoli destruction (pulmonary emphysema).

A histological examination shows that in the lungs of the control group, pulmonary alveolar macrophage infiltration into pulmonary alveoli of the right frontal lobe, intermediate lobe, and posterior lobe, inflammatory cell infiltration, mononuclear cell infiltration into pulmonary alveolar walls, blood vessels, and peribronchiolar regions, pulmonary alveolar wall thickening, and foreign-body granuloma inflammation were observed. These results confirmed that the control group developed the symptoms of COPD. On the other hand, single subcutaneous administration of Preparation Example 1 suppressed tissue changes of the pulmonary intermediate lobe and posterior lobe.

These results confirmed efficacy of single subcutaneous administration of Preparation Example 1 (10 mg/kg in terms of Compound 1) on airway resistance, functional residual capacity, residual volume increase, histological examination, and pulmonary alveoli destruction in COPD guinea pig models to which the cigarette smoke solution and LPS solution were endotracheally administered. The results of this efficacy test revealed that single intravenous administration of Preparation Example 1 at a dose of 1 mg/kg in terms of Compound 1 has efficacy equivalent to or higher than that achieved by single subcutaneous administration of Preparation Example 1 at a dose of 10 mg/kg in terms of Compound 1.

Pharmacological Effect Test 5: Investigation of Combined Effect of Ozagrel Hydrochloride with Compounds 3 and 7 in Pulmonary Hypertension Models

After Slc;Wistar female rats (5 weeks old) were anesthetized with inhaled isoflurane, an aqueous monocrotaline (MCT) solution was subcutaneously administered into the dorsal region of each rat at a dose of 60 mg/kg (an injection volume of 3 mL/Kg) using a disposable syringe and a 27G disposable injection needle to prepare pulmonary hypertension models.

From immediately after the MCT administration until Day 42, test substances were repeatedly administered orally twice a day as shown in the following table.

The test substances were suspended in 0.5% aqueous CMC-Na solutions, and the aqueous solutions were orally administered repeatedly at a dose of 5 mL/kg.

Table 7

| Group | Administered substance | Dose | Number of animals |
|---|---|---|---|
| 1 | 0.5 CMC-Na aqueous solution | 5 mL/kg | 20 |
| 2 | Compound 3 | 0.1 mg/kg x 2/day | 20 |
| 3 | Ozagrel Hydrochloride | 100 mg/kg x 2/day | 20 |
| 4 | Compound 3 + Ozagrel Hydrochloride | 0.1 mg/kg + 100 mg/kg/day | 20 |
| 5 | Compound 7 | 0.1 mg/kg x 2/day | 20 |
| 6 | Compound 7 + Ozagrel Hydrochloride | 0.1 mg/kg + 100 mg/kg/day | 20 |

A dose of 0.1 mg/kg, which is the oral dose of Compound 3 ($PGI_2$ agonist) and Compound 7 ($PGE_1$ derivative),

is the maximum tolerated dose that does not show blood-pressure-lowering effect. The dose of ozagrel hydrochloride ($TXA_2$ synthetase inhibitor, No.09702, LKT Laboratories, Inc.), 100 mg/kg, is the amount that completely inhibits the $TXA_2$ synthetase.

Table 10 shows the survival rate changes.

Results: When Compound 3 (Group 2), Compound 7 (Group 5), or ozagrel (Group 3) alone was administered to the pulmonary hypertension models, the survival rate curves of these groups were similar to that of the medium-administered group (Group 1). Thus, no survival prolongation effect was observed.

It has been reported that Compound 3 administered alone to an MCT-induced mild pulmonary hypertension model was efficacious and provided a synergistic effect with a PDEV inhibitor (sildenafil) (Am J Respir Crit Care Med 169: 34-38-2004). However, unlike the MCT-induced severe pulmonary hypertension model in the present invention, an evaluation using a mild disease model was reported. Furthermore, the synergetic effect reported is a combined effect with a PDEV inhibitor, which has an action mechanism different from ozagrel.

In contrast, combined administration of Compound 3 with ozagrel (Group 4) and combined administration of Compound 7 with ozagrel (Group 6) provided prolonged survival rates compared to the medium administration (Group 1) and administration of each of the compounds alone (Groups 2, 3, and 5). The results thus showed that the combined administration is effective.

A randomized double blind trial using Compound 3 was performed in Europe and the United States. Exercise tolerance was improved after a relatively short-term (after 12 weeks) (J Am Coll Cardiol 39: 1496-1502, 2002), but no significant difference was observed in long-term effects (after 1 year)(J Am Coll Cardiol 41: 2119-2125, 2003). These results revealed that long-term administration of beraprost (Compound 3) increased $TXA_2$ production capacity in blood, thus exhibiting tolerance.

These results revealed efficacy of a intravenous infusion (combination drug) combined $PGI_2$ (epoprostenol) with sodium ozagrel and a oral preparation (combination drug) combined ozagrel fydrochloride with Compound 3 (Beraprost) or Compound 7 (ornoprostol), which is a $PGE_1$ derivative.

Pharmacological Effect Test 6: Activity of Compound 1 to Promote Production of Various Cytokines and PGs

Normal human newborn foreskin dermal fibroblasts (Fibrocell NHDF (NB)) provided from Kurabo Industries, Ltd., were cultured to confluency at 5 x $10^6$ cells/mL/48 well plate (DMEM 10%FBS). Twenty-four hours after replacing the medium with (serum-free) DMEM, the medium was replaced with DMEM (0.2% FBS), and Compound 1 (1 $\mu$M) and a solvent (DMSO) was added. The cells were cultured for 72 hours (n = 3).

Cytokines in the culture were measured by ELISA, and prostaglandins were measured by EIA.

Table 8 shows the results. These results confirmed new activity to promote production of HMGB1 and PGs ($PGI_2$ and $PGE_2$) in addition to SDF-1, HGF, VEGF, and G-CSF.

Table 8

| Cytokine/PGs | Control (solvent) group; M $\pm$ S.E. | Compound 1 (1 $\mu$M); M $\pm$ S.E. |
|---|---|---|
| SDF-1$\alpha$ (pg/mL) | 1307.16 $\pm$ 29.665 | 2457.46 $\pm$ 231.884 |
| HGF (pg/mL) | 12.60 $\pm$ 0.508 | 102.60 $\pm$ 15.658 |
| HMGB1(ng/mL) | 0.743 $\pm$ 0.096 | 1.480 $\pm$ 0.147 |
| VEGF (pg/mL) | N.D. (10 pg/mL or less) | 38.44 $\pm$ 11.857 |
| G-CSF (pg/mL) | N.D. (20 pg/mL or less) | 28.71 $\pm$ 6.110 |
| 6-keto-$PGF_1\alpha$ ($PGI_2$) pg/mL | 16.3 $\pm$ 0.471 | 44.5 $\pm$ 4.014 |
| $PGE_2$ | 15.6 $\pm$ 1.825 | 38.1 $\pm$ 6.392 |

$PGI_2$ was measured by using a degradation product of $PGI_2$ (6-keto-$PGF_1\alpha$).

## Claims

1. A lung-specific therapeutic agent for use in the therapy of a pulmonary disease, wherein the therapeutic agent comprises sustained-release microspheres containing a pharmaceutical compound, wherein the microspheres have a number average particle size of 20 to 40 $\mu$m, and a maximum particle size of 100 $\mu$m or less, wherein the therapeutic agent is to be intravenously administered, wherein the pharmaceutical compound is a pulmonary disease therapeutic

drug.

2. The lung-specific therapeutic agent for use according to claim 1, wherein the pulmonary disease therapeutic drug is at least one member selected from the group consisting of beclomethasone, fluticasone, montelukast, tiotropium, imidafenacin, beraprost, carbacyclin, sivelestat, tulobuterol, salmeterol, and salts thereof.

3. The lung-specific therapeutic agent for use according to claim 1, wherein the pulmonary disease therapeutic drug is at least one in vivo regeneration factor selected from the group consisting of b-FGF, HGF, EGF, SDF-1, IGF-1, LIF, HMGB1, G-CSF, and extracellular matrices.

4. The lung-specific therapeutic agent for use according to claim 1, wherein the pulmonary disease therapeutic drug is a compound represented by Formula (I)

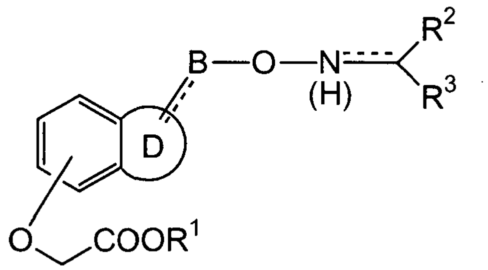

(I)

wherein

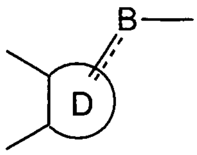

represents

(i) 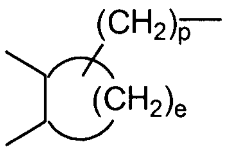 , (ii) 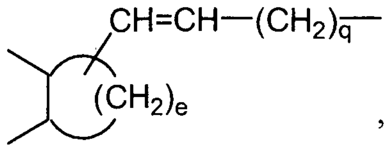 ,

(iii) 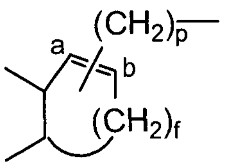 or (iv) 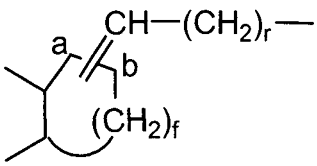 ,

wherein $R^1$ represents hydrogen or $C_{1-4}$ alkyl,
$R^2$ represents (i) hydrogen, (ii) $C_{1-8}$ alkyl, (iii) phenyl or $C_{4-7}$ cycloalkyl, (iv) a 4- to 7-membered monocycle containing one nitrogen atom, (v) a benzene ring- or $C_{4-7}$ cycloalkyl-substituted $C_{1-4}$ alkyl group, or (vi) a $C_{1-4}$ alkyl group substituted with a 4- to 7-membered monocycle containing one nitrogen atom,
$R^3$ represents (i) $C_{1-8}$ alkyl, (ii) phenyl or $C_{4-7}$ cycloalkyl, (iii) a 4- to 7-membered monocycle containing one nitrogen atom, (iv) a benzene ring- or $C_{4-7}$ cycloalkyl-substituted $C_{1-4}$ alkyl group, or (v) a $C_{1-4}$ alkyl group substituted with a 4- to 7-membered monocycle containing one nitrogen atom,
e represents an integer of 3 to 5,
f represents an integer of 1 to 3,
p represents an integer of 1 to 4,
q represents 1 or 2, and
r represents an integer of 1 to 3;

provided that when

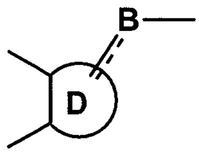

is a group defined in (iii) or (iv), each of $-(CH_2)_p-$ and $=CH-(CH_2)_s-$ bonds to either position a or b on the ring, and the rings in $R^2$ and $R^3$ may be substituted with one to three substituents selected from the group consisting of $C_{1-4}$ alkyl groups, $C_{1-4}$ alkoxy groups, halogen atoms, nitro groups, and trihalomethyl groups, or
a salt thereof.

**5.** The lung-specific therapeutic agent for use according to claim 4, wherein the pulmonary disease therapeutic drug is (E)-[5-[2-[1-phenyl-1-(3-pyridyl)methylideneaminooxy]ethyl]-7,8-dihydronaphthalen-1-yloxy]acetic acid.

**6.** The lung-specific therapeutic agent for use according to claim 1, wherein the pulmonary disease therapeutic drug is at least one member selected from the group consisting of (±)-(1R,2R,3aS,8bS)-2,3,3a,8b-tetrahydro-2-hydroxy-1-[(E)-(3S,4RS)-3-hydroxy-4-methyl-1-octen-6-inyl]-1H-cyclopenta[b]benzofuran-5-butanoic acid (beraprost), 2-{4-[N-(5,6-diphenylpyrazin-2-yl)-N-isopropylamino]butyloxy} acetic acid (MRE-269), carbacyclin, and
salts thereof.

**7.** The lung-specific therapeutic agent for use according to claim 1, wherein the sustained-release microspheres contain a biodegradable polymer selected from the group consisting of gelatin hydrogels, liposomes, lipids, polylactic acids, lactic acid-glycolic acid copolymers, polyglycolic acids, polydioxanes, and mixtures thereof.

**8.** The lung-specific therapeutic agent for use according to claim 1, which is a sustained-release microsphere preparation comprising a pulmonary disease therapeutic drug selected from the group consisting of beclomethasone, fluticasone, montelukast, tiotropium, imidafenacin, beraprost, carbacyclin, sivelestat, tulobuterol, salmeterol, and salts thereof; and at least one biodegradable polymer selected from the group consisting of gelatin hydrogels, liposomes, lipids, polylactic acids, lactic acid-glycolic acid copolymers, polyglycolic acids, polydioxanes, and mixtures thereof.

**9.** The lung-specific therapeutic agent for use according to claim 1, which comprises said sustained-release microsphere preparation containing at least one pulmonary disease therapeutic drug selected from the group consisting of (E)-[5-[2-[1-phenyl-1-(3-pyridyl)methylideneaminooxy]ethyl]-7,8-dihydronaphthalene-1-yloxy]acetic acid, (±)-(1R,2R,3aS,8bS)-2,3,3a,8b-tetrahydro-2-hydroxy-1-[(E)-(3S,4RS)-3-hydroxy-4-methyl-1-octene 6-inyl]-1H-cyclopenta[b]benzofuran-5-butanoic acid (beraprost), 2-{4-[N-(5,6-diphenylpyrazin-2-yl)-N-isopropylamino]butyloxy} acetic acid (MRE-269), and salts thereof; and a biodegradable polymer selected from the group consisting of polylactic acids, lactic acid-glycolic acid copolymers, and mixtures thereof.

**10.** The lung-specific therapeutic agent for use according to claim 1, which comprises sustained-release microspheres containing at least one pulmonary disease therapeutic drug selected from the group consisting of
beclomethasone, fluticasone, montelukast, tiotropium, imidafenacin, beraprost, carbacyclin, sivelestat, tulobuterol, salmeterol; (E)-[5-[2-[1-phenyl-1-(3-pyridyl)methylideneaminooxy]ethyl]-7,8-dihydronaphthalen-1-yloxy]acetic acid; (±)-(1R,2R,3aS,8bS)-2,3,3a,8b-tetrahydro-2-hydroxy-1-[(E)-(3S,4RS)-3-hydroxy-4-methyl-1-octene 6-inyl]-1H-cyclopenta[b]benzofuran-5-butanoic acid (beraprost); 2- {4-[N-(5,6-diphenylpyrazin-2-yl)-N-isopropylamino]butyloxy} acetic acid (MRE-269); and
salts thereof,
the therapeutic agent is to be administered at a dose of 6 mg/kg or less in terms of the sustained-release microspheres.

**11.** The lung-specific therapeutic agent for use according to any one of claims 1 to 10, wherein the pulmonary disease is at least one member selected from the group consisting of acute pneumonia, fibroid lung, interstitial pneumonia, pulmonary hypertension, chronic obstructive pulmonary diseases (COPD), chronic bronchitis, pulmonary emphysema, asthma, intractable asthma, systemic inflammatory response syndrome (SIRS), acute lung injury (ALI), respiratory distress syndrome (ARDS), sarcoidosis, chronic idiopathic pulmonary thromboembolism, diffuse panbronchiolitis, cystic fibrosis, hypersensitivity pneumonitis, lung cancer, obesity hypoventilation syndrome, alveolar hypoventilation syndrome, and chronic rejection in lung transplantation.

**Patentansprüche**

1. Ein lungenspezifisches therapeutisches Mittel zur Verwendung bei der Behandlung einer Lungenerkrankung, wobei das therapeutische Mittel Mikrokügelchen mit verzögerter Freisetzung umfasst, die eine pharmazeutische Verbindung enthalten, wobei die Mikrokügelchen ein Zahlenmittel der Teilchengröße von 20 bis 40 $\mu$m und eine maximale Teilchengröße von 100 $\mu$m oder weniger aufweisen, wobei das therapeutische Mittel intravenös zu verabreichen ist, wobei die pharmazeutische Verbindung ein Arzneistoff für eine Lungenerkrankung ist.

2. Das lungenspezifische therapeutische Mittel zur Verwendung gemäß Anspruch 1, wobei der Arzneistoff für eine Lungenerkrankung mindestens einer, ausgewählt aus der Gruppe bestehend aus Beclomethason, Fluticason, Montelukast, Tiotropium, Imidafenacin, Beraprost, Carbacyclin, Sivelestat, Tulobuterol, Salmeterol und Salzen davon, ist.

3. Das lungenspezifische therapeutische Mittel zur Verwendung gemäß Anspruch 1, wobei der Arzneistoff für eine Lungenerkrankung mindestens ein in vivo Regenerationsfaktor, ausgewählt aus der Gruppe bestehend aus b-FGF, HGF, EGF, SDF-1, IGF-1, LIF, HMGB1, G-CSF und extrazelluläre Matrices, ist.

4. Das lungenspezifische therapeutische Mittel zur Verwendung gemäß Anspruch 1, wobei der Arzneistoff für eine Lungenerkrankung eine durch die Formel (I) dargestellte Verbindung ist:

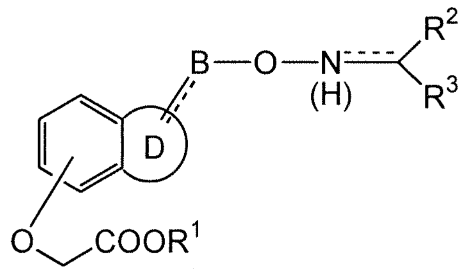

(I)

wobei

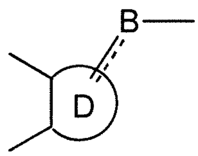

gleich

(i) 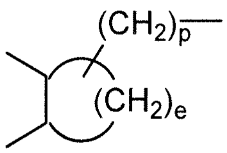 , (ii) 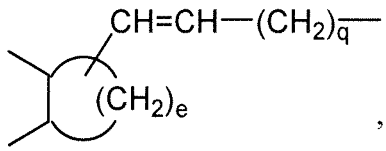 ,

(iii) 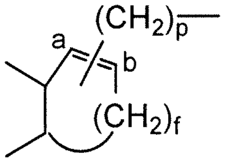 oder (iv) 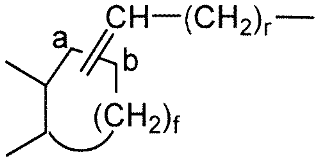 ,

ist,

wobei $R^1$ Wasserstoff oder $C_{1-4}$-Alkyl ist,
$R^2$ (i) Wasserstoff, (ii) $C_{1-8}$-Alkyl, (iii) Phenyl oder $C_{4-7}$-Cycloalkyl, (iv) ein 4- bis 7-gliedriger Monocyclus, der ein Stickstoffatom enthält, (v) eine mit einem Benzolring oder $C_{4-7}$-Cycloalkyl substituierte $C_{1-4}$-Alkylgruppe oder (vi) eine $C_{1-4}$-Alkylgruppe, die mit einem 4- bis 7-gliedrigen Monocyclus, der ein Stickstoffatom enthält,

substituiert ist, ist,
$R^3$ (i) $C_{1-8}$-Alkyl, (ii) Phenyl oder $C_{4-7}$-Cycloalkyl, (iii) ein 4- bis 7-gliedriger Monocyclus, der ein Stickstoffatom enthält, (iv) eine mit einem Benzolring oder $C_{4-7}$-Cycloalkyl substituierte $C_{1-4}$-Alkylgruppe oder (v) eine $C_{1-4}$-Alkylgruppe, die mit einem 4- bis 7-gliedrigen Monocyclus, der ein Stickstoffatom enthält, substituiert ist, ist,
e eine ganze Zahl von 3 bis 5 ist,
f eine ganze Zahl von 1 bis 3 ist,
p eine ganze Zahl von 1 bis 4 ist,
q gleich 1 oder 2 ist, und
r eine ganze Zahl von 1 bis 3 ist,

vorausgesetzt, dass wenn

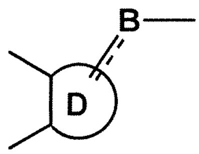

eine in (iii) oder (iv) definierte Gruppe ist, $-(CH_2)_p-$ und $=CH-(CH_2)_s-$ jeweils entweder an Position a oder b des Rings gebunden sind, und die Ringe in $R^2$ und $R^3$ mit einem bis drei Substituenten, ausgewählt aus der Gruppe bestehend aus $C_{1-4}$-Alkylgruppen, $C_{1-4}$-Alkoxygruppen, Halogenatomen, Nitrogruppen und Trihalogenmethylgruppen, substituiert sein können, oder ein Salz davon.

5. Das lungenspezifische therapeutische Mittel zur Verwendung gemäß Anspruch 4, wobei der Arzneistoff für eine Lungenerkrankung (E)-[5-[2-[1-Phenyl-1-(3-pyridyl)methylidenaminooxy]ethyl]-7,8-dihydronaphthalin-1-yloxy]essigsäure ist.

6. Das lungenspezifische therapeutische Mittel zur Verwendung gemäß Anspruch 1, wobei der Arzneistoff für eine Lungenerkrankung mindestens einer, ausgewählt aus der Gruppe bestehend aus (±)-(1R,2R,3aS,8bS)-2,3,3a,8b-Tetrahydro-2-hydroxy-1-[(E)-(3S,4RS)-3-hydroxy-4-methyl-1-octen-6-inyl]-1H-cyclopenta[b]benzofuran-5-butansäure (Beraprost), 2-{4-[N-(5,6-Diphenylpyrazin-2-yl)-N-isopropylamino]butyloxy}essigsäure (MRE-269), Carbacyclin, und Salzen davon, ist.

7. Das lungenspezifische therapeutische Mittel zur Verwendung gemäß Anspruch 1, wobei die Mikrokügelchen mit verzögerter Freisetzung ein biologisch abbaubares Polymer, ausgewählt aus der Gruppe bestehend aus Gelatine-Hydrogelen, Liposomen, Lipiden, Polymilchsäuren, Milchsäure-Glycolsäure-Copolymeren, Polyglycolsäuren, Polydioxanen und Gemischen davon, enthalten.

8. Das lungenspezifische therapeutische Mittel zur Verwendung gemäß Anspruch 1, bei dem es sich um ein Mikrokügelchenpräparat mit verzögerter Freisetzung handelt, das einen Arzneistoff für eine Lungenerkrankung, ausgewählt aus der Gruppe bestehend aus Beclomethason, Fluticason, Montelukast, Tiotropium, Imidafenacin, Beraprost, Carbacyclin, Sivelestat, Tulobuterol, Salmeterol und Salzen davon; und mindestens ein biologisch abbaubares Polymer, ausgewählt aus der Gruppe bestehend aus Gelatine-Hydrogelen, Liposomen, Lipiden, Polymilchsäuren, Milchsäure-Glykolsäure-Copolymeren, Polyglycolsäuren, Polydioxanen und Gemischen davon, umfasst.

9. Das lungenspezifische therapeutische Mittel zur Verwendung gemäß Anspruch 1, welches das Mikrokügelchenpräparat mit verzögerter Freisetzung umfasst, das mindestens einen Arzneistoff für eine Lungenerkrankung, ausgewählt aus der Gruppe bestehend aus (E)-[5-[2-[1-Phenyl-1-(3-pyridyl)methylidenaminooxy]ethyl]-7,8-dihydronaphthalin-1-yloxy]essigsäure, (±)-(1R,2R,3aS,8bS)-2,3,3a,8b-Tetrahydro-2-hydroxy-1-[(E)-(3 S,4RS)-3-hydroxy-4-methyl-1-octen-6-inyl]-1H-cyclopenta[b]benzofuran-5-butansäure (Beraprost), 2-{4-[N-(5,6-Diphenylpyrazin-2-yl)-N-isopropylamino]butyloxy}essigsäure (MRE-269) und Salzen davon; und ein biologisch abbaubares Polymer, ausgewählt aus der Gruppe bestehend aus Polymilchsäuren, Milchsäure-Glycolsäure-Copolymeren und Gemischen davon, enthält.

10. Das lungenspezifische therapeutische Mittel zur Verwendung gemäß Anspruch 1, das Mikrokügelchen mit verzögerter Freisetzung umfasst, die mindestens einen Arzneistoff für eine Lungenerkrankung, ausgewählt aus der Gruppe bestehend aus Beclomethason, Fluticason, Montelukast, Tiotropium, Imidafenacin, Beraprost, Carbacyclin, Sivelestat, Tulobuterol, Salmeterol; (E)-[5-[2-[1-Phenyl-1-(3-pyridyl)methylidenaminooxy]ethyl]-7,8-dihydronaphthalin-1-yloxy]essigsäure; (±)-(1R,2R,3aS,8bS)-2,3,3a,8b-Tetrahydro-2-hydroxy-1-[(E)-(3S,4RS)-3-hydroxy-4-methyl-1-octen-6-inyl]-1H-cyclopenta[b]benzofuran-5-butansäure (Beraprost); 2-{4-[N-(5,6-Diphenylpyrazin-2-yl)-N-isopropylamino]butyloxy}essigsäure (MRE-269); und
Salzen davon, umfasst,
wobei das therapeutische Mittel in einer Dosis von 6 mg/kg oder weniger, bezogen auf die Mikrokügelchen mit verzögerter Freisetzung, zu verabreichen ist.

**11.** Das lungenspezifische therapeutische Mittel zur Verwendung gemäß einem der Ansprüche 1 bis 10, wobei die Lungenerkrankung mindestens eine, ausgewählt aus der Gruppe bestehend aus akuter Lungenentzündung, Fibroid-Lunge, interstitieller Lungenentzündung, pulmonaler Hypertonie, chronisch obstruktiven Lungenerkrankungen (COPD), chronischer Bronchitis, Lungenemphysem, Asthma, hartnäckigem Asthma, systemischem inflammatorischem Antwortsyndrom (SIRS), akuter Lungenschädigung (ALI), Atemnotsyndrom (ARDS), Sarkoidose, chronischem idiopathischem pulmonalem Thromboembolismus, diffuser Panbronchiolitis, zystischer Fibrose, hypersensitive Pneumonitis, Lungenkrebs, Adipositas-Hypoventilationssyndrom, alveolarem Hypoventilationssyndrom und chronischer Abstoßung bei Lungentransplantation, ist.

## Revendications

**1.** Agent thérapeutique spécifique aux poumons pour utilisation dans le traitement d'une maladie pulmonaire, dans lequel l'agent thérapeutique comprend des microsphères à libération prolongée contenant un composé pharmaceutique, dans lequel les microsphères ont une taille de particule moyenne en nombre de 20 à 40 μm, et une taille de particule maximale de 100 μm ou moins, dans lequel l'agent thérapeutique doit être administré par voie intraveineuse, dans lequel le composé pharmaceutique est un médicament thérapeutique pour maladie pulmonaire.

**2.** Agent thérapeutique spécifique aux poumons pour utilisation selon la revendication 1, dans lequel le médicament thérapeutique pour maladie pulmonaire est au moins un élément choisi dans le groupe constitué de béclométasone, fluticasone, montélukast, tiotropium, imidafénacine, béraprost, carbacycline, sivélestat, tulobutérol, salmétérol et sels de ceux-ci.

**3.** Agent thérapeutique spécifique aux poumons pour utilisation selon la revendication 1, dans lequel le médicament thérapeutique pour maladie pulmonaire est au moins un facteur de régénération in vivo choisi dans le groupe constitué de b-FGF, HGF, EGF, SDF-1, IGF-1, LIF, HMGB1, G-CSF et de matrices extracellulaires.

**4.** Agent thérapeutique spécifique aux poumons pour utilisation selon la revendication 1, dans lequel le médicament thérapeutique pour maladie pulmonaire est un composé représenté par la formule (I)

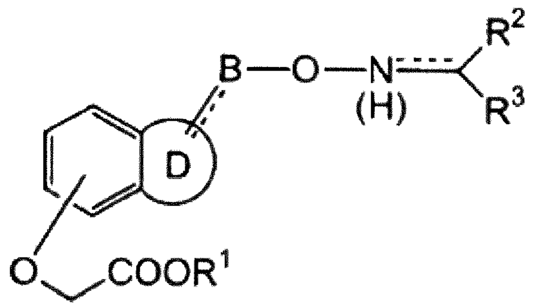

(I)

dans lequel

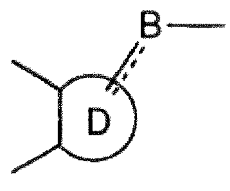

représente

(i) 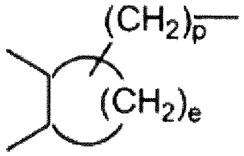 , (ii) 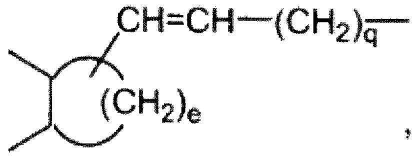 ,

(iii) 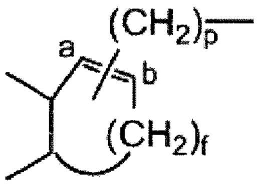 ou (iv) 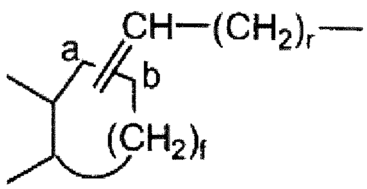

dans lequel $R^1$ représente un hydrogène ou un alkyle en $C_1$-$C_4$,
$R^2$ représente (i) un d'hydrogène, (ii) un alkyle en $C_1$-$C_8$, (iii) un phényle ou cycloalkyle en $C_4$-$C_7$, (iv) un monocycle à 4 ou 7 éléments contenant un atome d'azote, (v) un groupe alkyle en $C_1$-$C_4$ substitué par un cycle benzène ou un groupe cycloalkyle en $C_4$-$C_7$ ou (vi) un groupe alkyle en $C_1$-$C_4$ substitué par un monocycle à 4 ou 7 éléments contenant un atome d'azote,
$R^3$ représente (i) un alkyle en $C_1$-$C_8$, (ii) un phényle ou cycloalkyle en $C_4$-$C_7$, (iii) un monocycle à 4 ou 7 éléments contenant un atome d'azote, (iv) un groupe alkyle en $C_1$-$C_4$ substitué par un cycle benzène ou un groupe cycloalkyle en $C_4$-$C_7$ ou (v) un groupe alkyle en $C_1$-$C_4$ substitué par un monocycle à 4 ou 7 éléments contenant un atome d'azote,
e représente un entier de 3 à 5,
f représente un entier de 1 à 3,
p représente un entier de 1 à 4,
q représente 1 ou 2, et
r représente un entier de 1 à 3 ;

à condition que lorsque

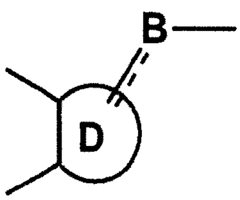

est un groupe défini dans (iii) ou (iv), chacun de $-(CH_2)_p-$ et $=CH-(CH_2)_s-$ se lie à l'une ou l'autre position de a ou b sur le cycle, et les cycles dans $R^2$ et $R^3$ peuvent être substitués par un à trois substituants choisis dans le groupe constitué de groupes alkyle en $C_1$-$C_4$, groupes alcoxy en $C_1$-$C_4$, atomes d'halogène, groupes nitro et groupes trihalométhyle, ou
un sel de ceux-ci.

**5.** Agent thérapeutique spécifique aux poumons pour utilisation selon la revendication 4, dans lequel le médicament thérapeutique pour maladie pulmonaire est de l'acide (E)-[5-[2-[1-phényl-1-(3-pyridyl)méthylidèneaminooxy]éthyl]-7,8-dihydronaphtalèn-1-yloxy]acétique.

**6.** Agent thérapeutique spécifique aux poumons pour utilisation selon la revendication 1, dans lequel le médicament thérapeutique pour maladie pulmonaire est au moins un élément choisi dans le groupe constitué d'acide (±)-(1R,2R,3aS,8bS)-2,3,3a,8b-tétrahydro-2-hydroxy-1-[(E)-(3S,4RS)-3-hydroxy-4-méthyl-1-octén-6-inyl]-1H-cyclopenta[b]benzofuran-5-butanoïque (béraprost), d'acide 2-{4-[N-(5,6-diphénylpyrazin-2-yl)-N-isopropylamino]butyloxy}acétique (MRE-269), carbacycline, et
des sels de ceux-ci.

**7.** Agent thérapeutique spécifique aux poumons pour utilisation selon la revendication 1, dans lequel les microsphères

à libération prolongée contiennent un polymère biodégradable choisi dans le groupe constitué d'hydrogels de gélatine, liposomes, lipides, acides polylactiques, copolymères d'acide lactique-acide glycolique, acides polyglycoliques, polydioxanes et mélanges de ceux-ci.

**8.** Agent thérapeutique spécifique aux poumons pour utilisation selon la revendication 1, qui est une préparation de microsphères à libération prolongée comprenant un médicament thérapeutique pour maladie pulmonaire choisi dans le groupe constitué de béclométhasone, fluticasone, montélukast, tiotropium, imidafénacine, béraprost, carbacycline, sivélestat, tulobutérol, salmétérol et de sels de ceux-ci ; et au moins un polymère biodégradable choisi dans le groupe constitué d'hydrogels de gélatine, liposomes, lipides, acides polylactiques, copolymères d'acide lactique-acide glycolique, acides polyglycoliques, polydioxanes et mélanges de ceux-ci.

**9.** Agent thérapeutique spécifique aux poumons pour utilisation selon la revendication 1, qui comprend ladite préparation de microsphères à libération prolongée contenant au moins un médicament thérapeutique pour maladie pulmonaire choisi dans le groupe constitué d'acide (E)-[5-[2-[1-phényl-1-(3-pyridyl)méthylidèneaminooxy]éthyl]-7,8-dihydronaphtalèn-1-yloxy]acétique, d'acide (±)-(1R,2R,3aS,8bS)-2,3,3a,8b-tétrahydro-2-hydroxy-1-[(E)-(3S,4RS)-3-hydroxy-4-méthyl-1-octén-6-inyl]-1H-cyclopenta[b]benzofuran-5-butanoïque (béraprost), d'acide 2-{4-[N-(5,6-diphénylpyrazin-2-yl)-N-isopropylamino]butyloxy}acétique (MRE-269), et de sels de ceux-ci ; et un polymère biodégradable choisi dans le groupe constitué d'acides polylactiques, de copolymères d'acide lactique-acide glycolique et de mélanges de ceux-ci.

**10.** Agent thérapeutique spécifique aux poumons pour utilisation selon la revendication 1, qui comprend des microsphères à libération prolongée contenant au moins un médicament thérapeutique pour maladie pulmonaire choisi dans le groupe constitué de
béclométhasone, fluticasone, montélukast, tiotropium, imidafénacine, béraprost, carbacycline, sivélestat, tulobutérol, salmétérol ;
acide (E)-[5-[2-[1-phényl-1-(3-pyridyl)méthylidèneaminooxy]éthyl]-7,8-dihydronaphtalèn-1-yloxy]acétique,
acide (±)-(1R,2R,3aS,8bS)-2,3,3a,8b-tétrahydro-2-hydroxy-1-[(E)-(3S,4RS)-3-hydroxy-4-méthyl-1-octén-6-inyl]-1H-cyclopenta[b]benzofuran-5-butanoïque (béraprost) ;
acide 2-{4-[N-(5,6-diphénylpyrazin-2-yl)-N-isopropylamino]butyloxy}acétique (MRE-269) ;
et de sels de ceux-ci,
l'agent thérapeutique doit être administré à une dose de 6 mg/kg ou moins en termes des microsphères à libération prolongée.

**11.** Agent thérapeutique spécifique aux poumons pour utilisation selon l'une quelconque des revendications 1 à 10, dans lequel la maladie pulmonaire est au moins un élément choisi dans le groupe constitué d'une pneumonie aiguë, d'un poumon fibreux, d'une pneumonie interstitielle, d'une hypertension pulmonaire, de bronchopneumopathies chroniques obstructives (BPCO), d'une bronchite chronique, d'un emphysème pulmonaire, de l'asthme, d'une asthme intraitable, d'un syndrome de réponse inflammatoire systémique (SIRS), d'une lésion pulmonaire aiguë (LPA), d'un syndrome de détresse respiratoire (ARDS), d'une sarcoïdose, d'une thrombo-embolie pulmonaire idiopathique chronique, d'une panbronchiolite diffuse, d'une fibrose cystique, d'une pneumopathie d'hypersensibilité, d'un cancer du poumon, d'un syndrome d'hypoventilation lié à l'obésité, d'un syndrome d'hypoventilation alvéolaire et d'un rejet chronique dans une greffe de poumon.

Fig. 1

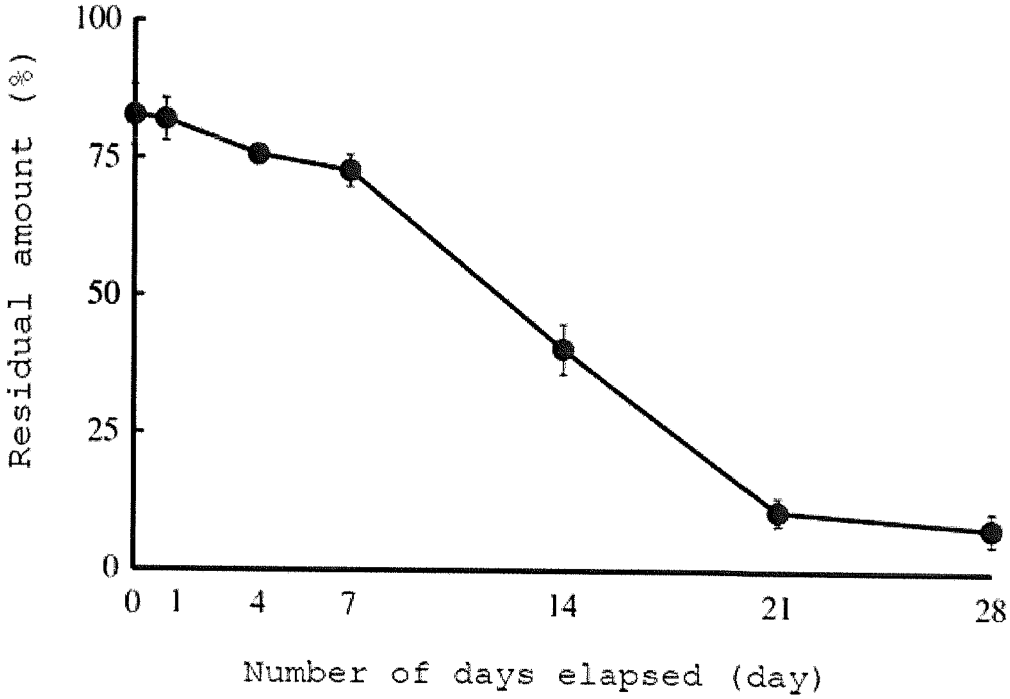
100
75
50
25
0
Residual amount (%)
0 1 4 7 14 21 28
Number of days elapsed (day)

Fig. 2

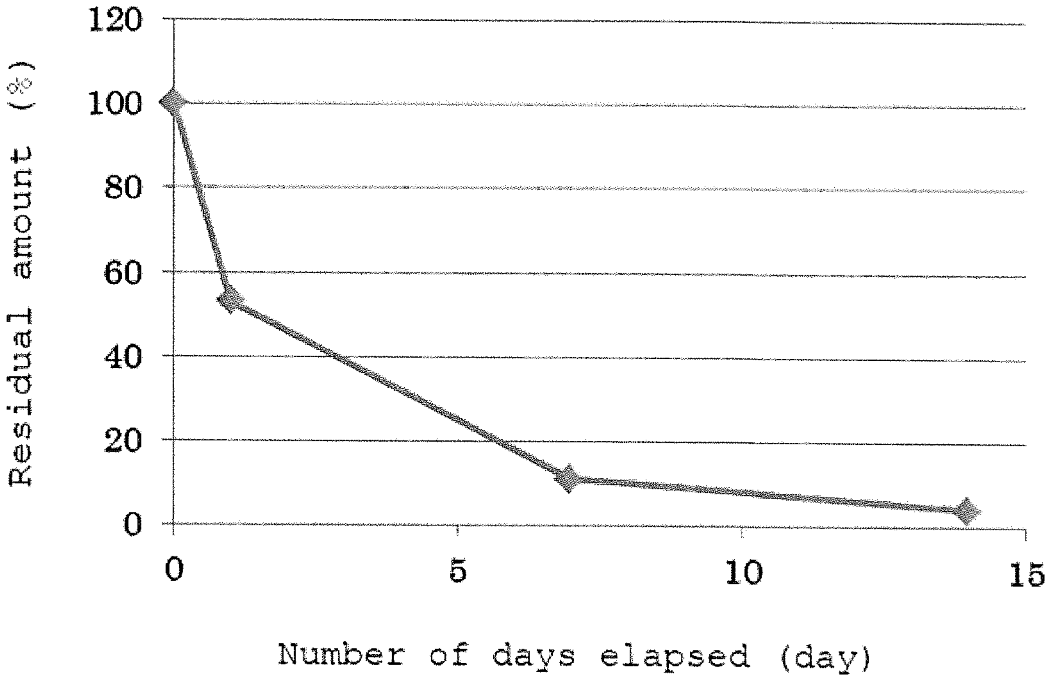
Residual amount (%)
120
100
80
60
40
20
0
0
5
10
15
Number of days elapsed (day)

Fig. 3

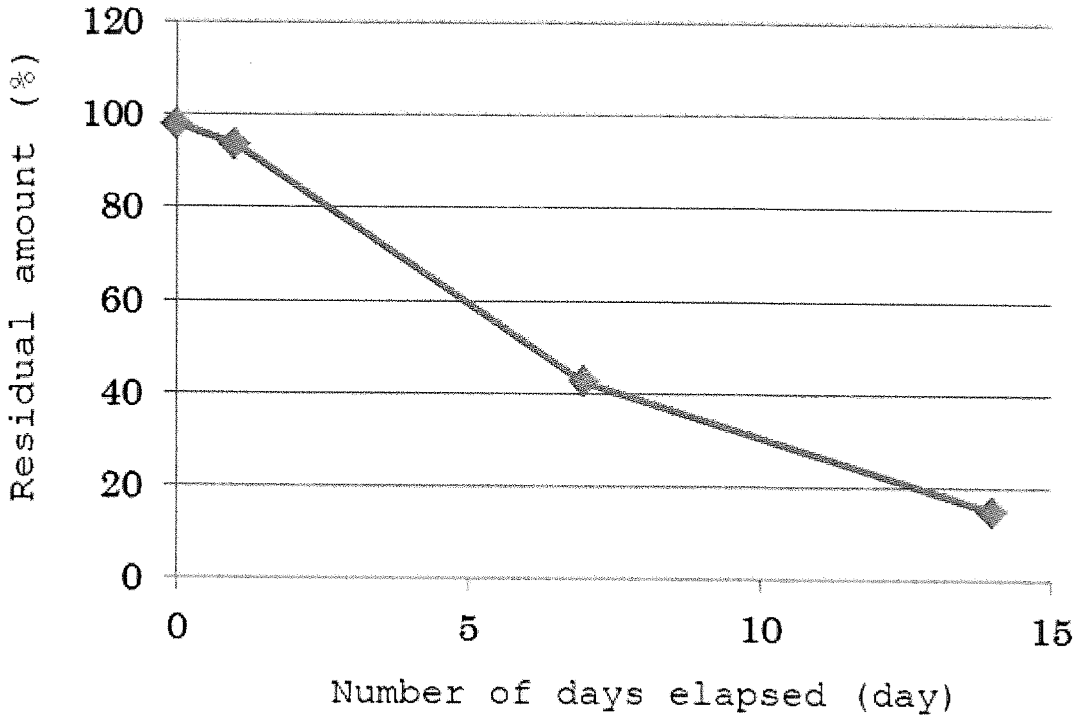
Residual amount (%)
120
100
80
60
40
20
0
0
5
10
15
Number of days elapsed (day)

Fig. 4

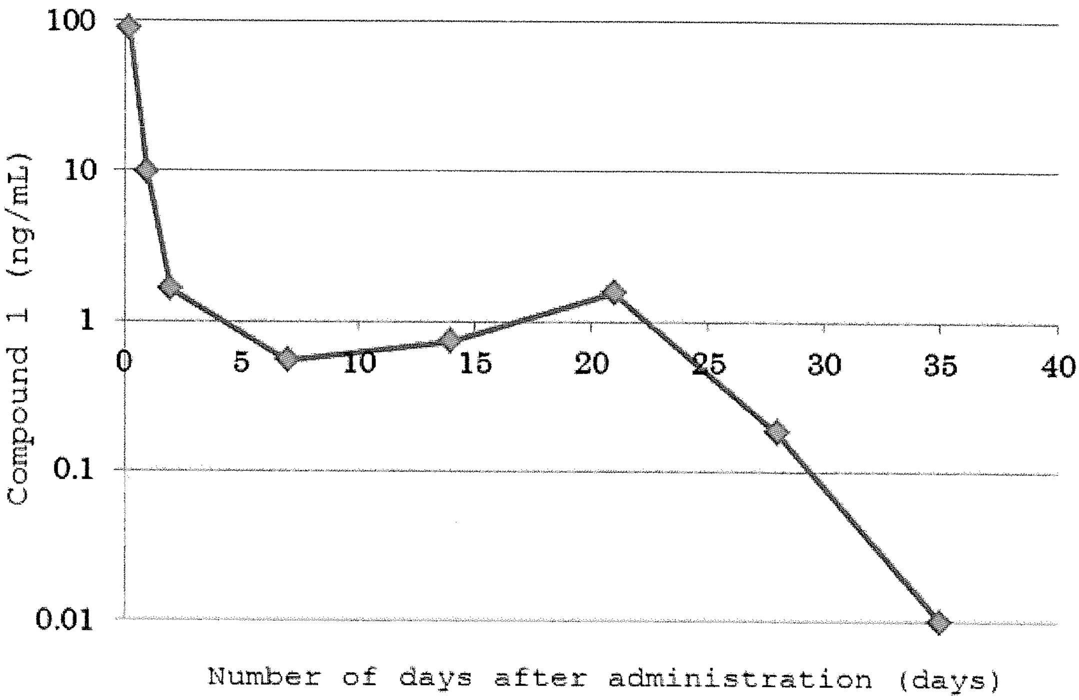
100
10
1
0.1
0.01
Compound 1 (ng/mL)
0
5
10
15
20
25
30
35
40
Number of days after administration (days)

Fig. 5

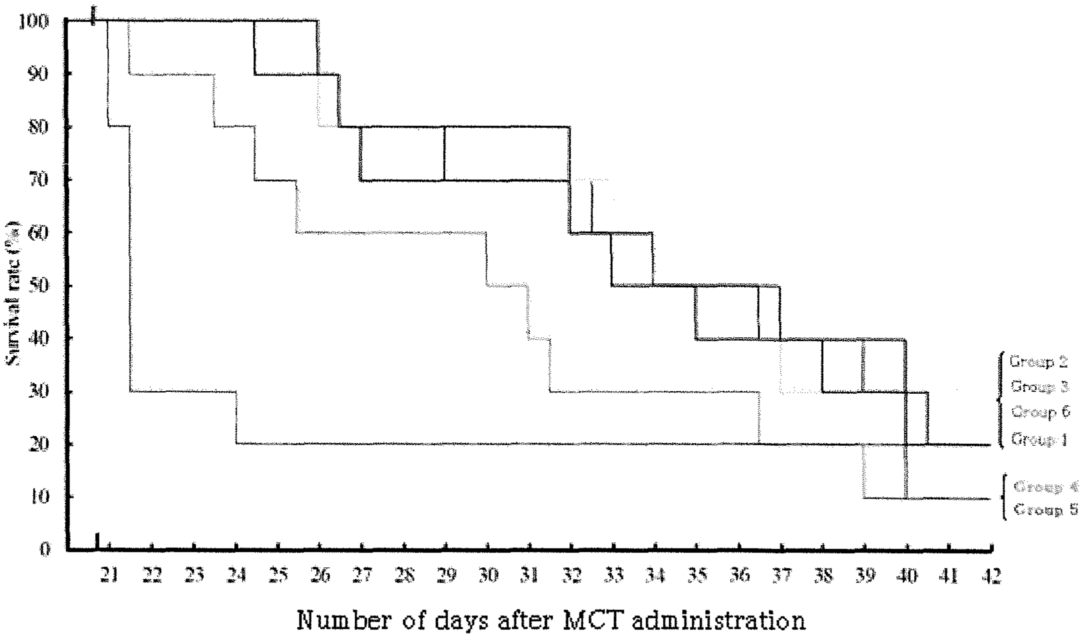
Survival rate (%)
100
90
80
70
60
50
40
30
20
10
0
21 22 23 24 25 26 27 28 29 30 31 32 33 34 35 36 37 38 39 40 41 42
Number of days after MCT administration
Group 2
Group 3
Group 6
Group 1
Group 4
Group 5

Fig. 6

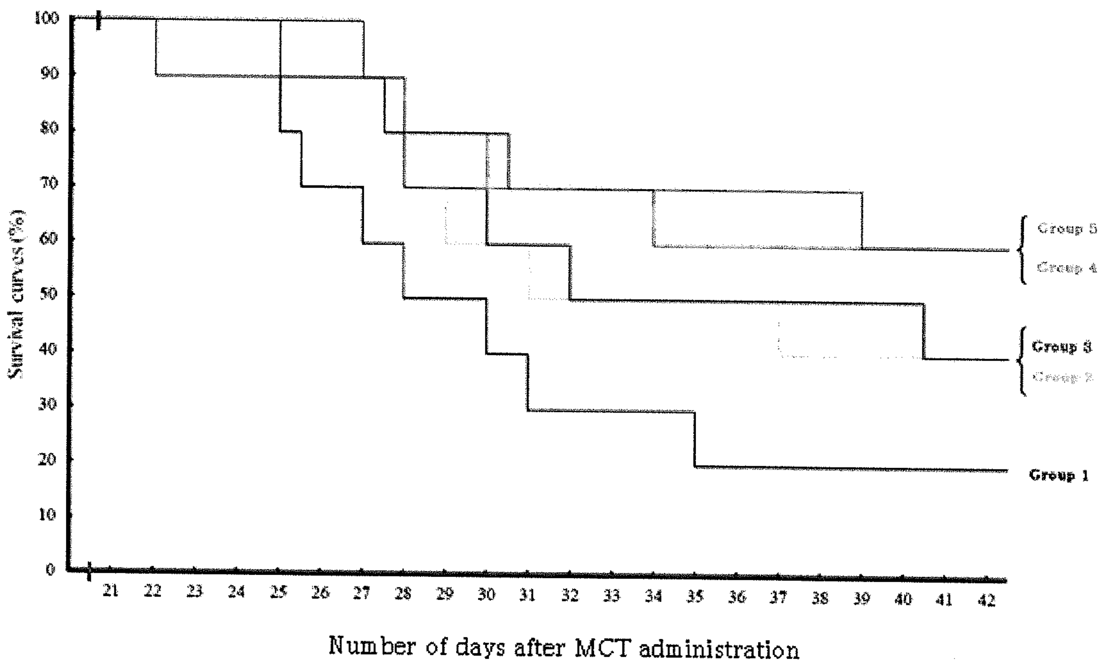
Survival curves (%)
100
90
80
70
60
50
40
30
20
10
0
21 22 23 24 25 26 27 28 29 30 31 32 33 34 35 36 37 38 39 40 41 42
Number of days after MCT administration
Group 5
Group 4
Group 3
Group 2
Group 1

Fig. 7

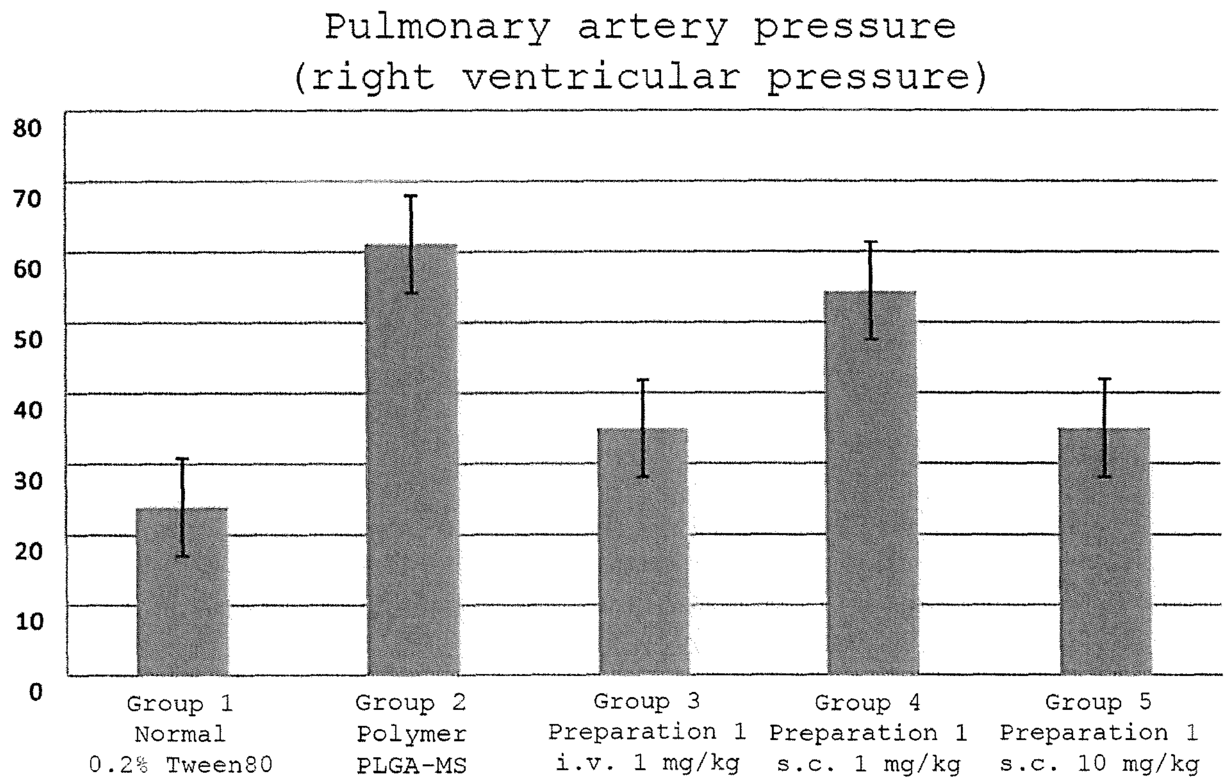
Pulmonary artery pressure
(right ventricular pressure)
80
70
60
50
40
30
20
10
0
Group 1
Normal
0.2% Tween80
Group 2
Polymer
PLGA-MS
Group 3
Preparation 1
i.v. 1 mg/kg
Group 4
Preparation 1
s.c. 1 mg/kg
Group 5
Preparation 1
s.c. 10 mg/kg

Fig. 8

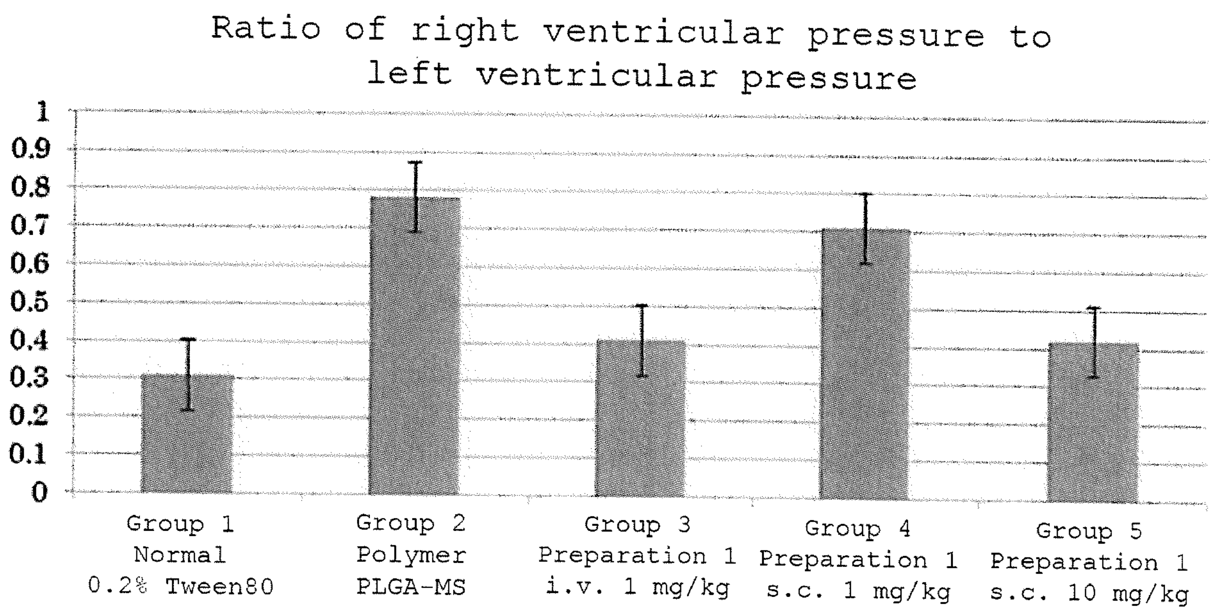
Ratio of right ventricular pressure to left ventricular pressure
1
0.9
0.8
0.7
0.6
0.5
0.4
0.3
0.2
0.1
0
Group 1
Normal
0.2% Tween80
Group 2
Polymer
PLGA-MS
Group 3
Preparation 1
i.v. 1 mg/kg
Group 4
Preparation 1
s.c. 1 mg/kg
Group 5
Preparation 1
s.c. 10 mg/kg

Fig. 9

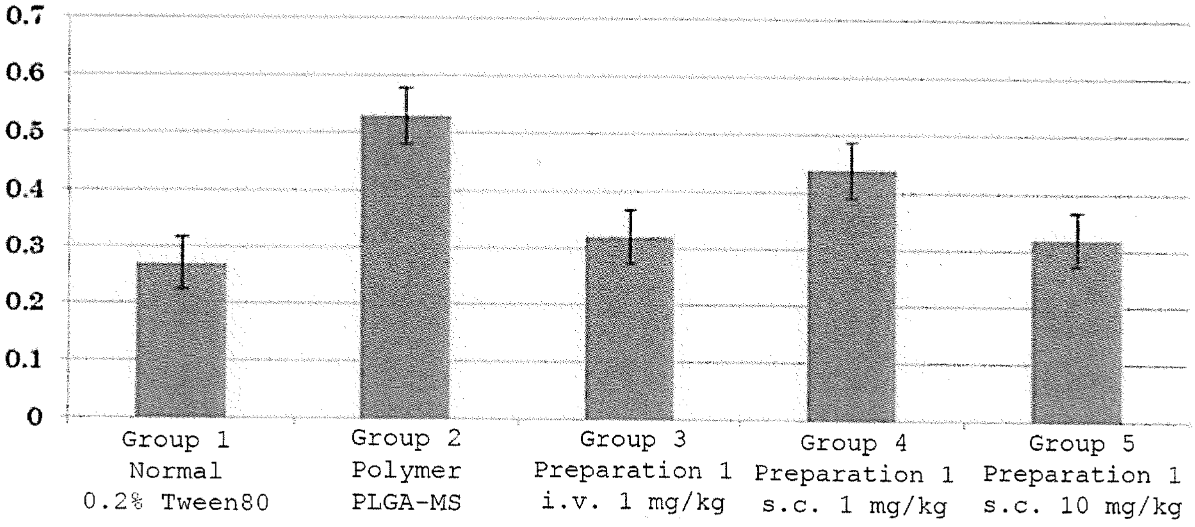
0.7
0.6
0.5
0.4
0.3
0.2
0.1
0
Group 1
Normal
0.2% Tween80
Group 2
Polymer
PLGA-MS
Group 3
Preparation 1
i.v. 1 mg/kg
Group 4
Preparation 1
s.c. 1 mg/kg
Group 5
Preparation 1
s.c. 10 mg/kg

Fig. 10

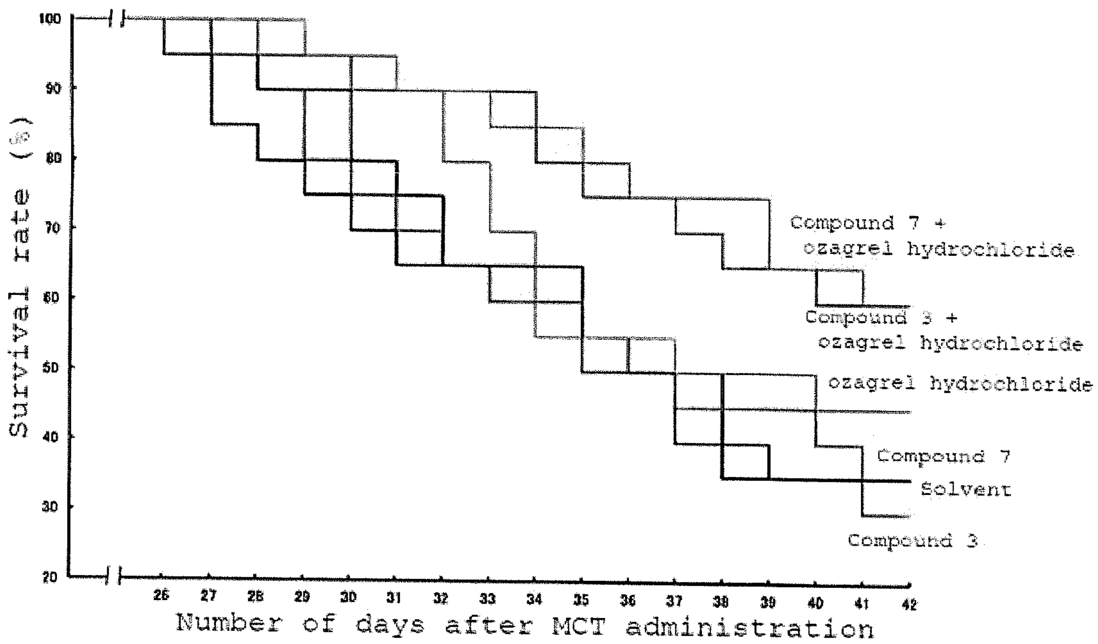
Survival rate (%)
100
90
80
70
60
50
40
30
20
26
27
28
29
30
31
32
33
34
35
36
37
38
39
40
41
42
Number of days after MCT administration
Compound 7 + ozagrel hydrochloride
Compound 3 + ozagrel hydrochloride
ozagrel hydrochloride
Compound 7
Solvent
Compound 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004032965 A
- WO 20080478631 A
- JP 2012208799 A
- WO 2008047863 A
- EP 2087890 A1
- JP 2004115413 A
- JP 4459543 B
- JP 4685090 B
- JP 2008137975 A
- JP H0741432 A
- JP 2011516443 A
- JP 2004091450 A
- JP 2005206491 A
- EP 0860430 A
- US 6110969 A
- WO 9933794 A
- EP 974580 A
- WO 9519964 A
- WO 9828264 A
- WO 9919300 A
- EP 0911321 A
- WO 9858911 A
- US 5698598 A
- US 6376533 B
- US 4132738 A
- US 3965143 A
- WO 0003980 A
- WO 9902164 A
- WO 0016760 A
- WO 0018744 A
- WO 0021542 A
- WO 0038663 A
- WO 0038690 A
- WO 0038667 A
- WO 0040248 A
- WO 0054808 A
- WO 0054809 A
- WO 0110426 A
- EP 1110949 A
- EP 1121939 A
- EP 1132086 A
- WO 200172268 A
- JP 2002104939 A
- WO 0242268 A
- JP 2002179595 A
- WO 0247669 A
- WO 0264564 A
- WO 03035064 A
- WO 03053923 A
- US 6552067 B
- US 5480998 A
- WO 1996026721 A
- WO 02088084 A
- WO 4497320 A


### Non-patent literature cited in the description


- *Am J Respir Crit Care Med,* 2008, vol. 177, 195-201
- *Am J Physiol Gastrointest Liver Physiol,* 2012, vol. 302, G420-G429
- *Cell,* 2011, vol. 43 (10), 26-35
- *Cell,* 2012, vol. 44 (2), 34-40
- *Biochem. Biophys. Res. Comm,* 1984, vol. 122, 145-1459
- *Development,* 1998, vol. 125, 1315-1324
- *Am J Respir Crit Care Med,* 2000, vol. 162, 707-715
- *Circulation,* 2004, vol. 110, 2896-2902
- *Am J Respir Cell Mol Biol,* 2002, vol. 26, 525-533
- *Circulation,* 2005, vol. 111, 1407-1414
- *Am J Respir Cell Med Bio,* 2005, vol. 32, 268-280
- *Nature Medicine,* 1999, vol. 5, 226-230
- *Am J Respir Crit Care Med,* 1997, vol. 156, 1937-1944
- *Am J Respir Crit Care Med,* 2005, vol. 172, 1575-1580
- *Circ J,* 2013, vol. 77, 2127-2133
- *Am J Physiol Lung Cell Mol Physiol,* 2006, vol. 290, L59-L65
- Annual Congress. European Respiratory Society, 2010
- *Clinical & Experimental Allergy,* 2009, vol. 40, 317-326
- *Am J Respir Cell Mol Biol,* 2012, vol. 47 (2), 170-177
- *The 60th Fall Meeting of the Japanese Society of Allergology,* 2010
- International Conference. American Thoracic Society, 2011
- *Arch Biochem Biophys,* 1981, vol. 212, 196

- *Adv Drug Derliv Rev,* 1998, vol. 31, 287-301
- *JR Soc Interface,* 2009, vol. 6 (3), S311-S324
- *J Biomater Sci Polym Edn,* 2001, vol. 12, 77-88
- *Drug Deliv Syst,* 2008, vol. 23 (3), 394
- *General Thoracic and Cardiovascular Surgery,* 2003, vol. 51, 281
- *PLoS ONE,* 2013, vol. 8 (7), e69302
- *Biomedicine & Aging Pathology,* 2011, vol. 1, 90-96
- *Clinical Science,* 2007, vol. 112, 607-616
- Research Project on Measures for Intractable Diseases. Japanese Ministry of Health, Labor and Welfare, 2012
- *Nuclear medicine,* 1979, vol. 16, 927-931
- *Biol Pharm. Bull.,* 2009, vol. 32 (9), 1559-1564
- *Am J Respir Crit Care Med,* 2004, vol. 169, 34-38
- *J Am Coll Cardiol,* 2002, vol. 39, 1496-1502
- *J Am Coll Cardiol,* 2003, vol. 41, 2119-2125